(19) Europäisches Patentamt
European Patent Office
Office européen des brevets

(11) Publication number: **0 497 740 B1**

(12) **EUROPEAN PATENT SPECIFICATION**

(45) Date of publication of patent specification: **14.12.94**

(51) Int. Cl.5: **C07C 69/92**, C07C 65/05, C07C 235/60, C07C 255/54, C07C 67/31, A61K 31/235, A61K 31/16, A61K 31/275, A61K 31/19

(21) Application number: **92810066.8**

(22) Date of filing: **29.01.92**

(54) **Benzyloxyphenyl derivatives.**

(30) Priority: **01.02.91 DE 4103003**
**30.03.91 DE 4110478**

(43) Date of publication of application:
**05.08.92 Bulletin 92/32**

(45) Publication of the grant of the patent:
**14.12.94 Bulletin 94/50**

(84) Designated Contracting States:
**AT BE CH DE DK ES FR GB GR IT LI LU NL PT SE**

(56) References cited:

**No relevant documents disclosed**

(73) Proprietor: **SANDOZ LTD.**
**Lichtstrasse 35**
**CH-4002 Basel (CH)**
(84) Designated Contracting States:
**BE CH DK ES FR GB GR IT LI LU NL PT SE**

(73) Proprietor: **SANDOZ-PATENT-GMBH**
**Humboldtstrasse 3**
**D-79539 Lörrach (DE)**
(84) Designated Contracting States:
**DE**

(73) Proprietor: **SANDOZ ERFINDUNGEN VERWALTUNGSGESELLSCHAFT M.B.H.**
**Brunner Strasse 59**
**A-1235 Vienna (AT)**
(84) Designated Contracting States:
**AT**

(72) Inventor: **Nussbaumer, Peter**
**Franz-Josef-Strasse 81/3**
**A-2344 Maria-Enzersdorf (AT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

The invention relates to the field of benzyloxyphenyl derivatives. It concerns compounds of formula I

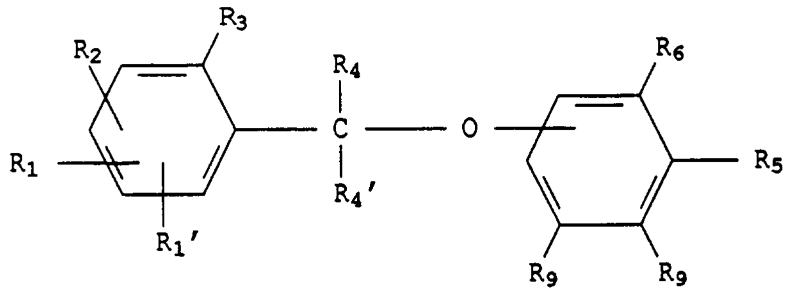

I

wherein
either

$R_1$, $R_2$ and $R_3$ independently are hydrogen, hydroxy, alkoxy, acyloxy, halogen or benzyloxy, whereby two of these substituents in adjacent positions together with the carbon atoms to which they are attached additionally can form an aliphatic, heteroaliphatic, aromatic or heteroaromatic ring and

$R_1'$ is hydrogen

or

$R_1$ and $R_1'$ in adjacent positions together with the carbon atoms to which they are attached form an aromatic ring and

$R_2$ and $R_3$ are as defined above,

either

$R_4$ and $R_4'$ independently are hydrogen, alkyl, alkenyl, cyano, carboxy, alkoxycarbonyl, $-CONR_7R_8$ or optionally hydroxy-, alkoxy- or halogen-substituted phenyl, whereby

$R_7$ and $R_8$ either independently are hydrogen, alkyl, phenyl or benzyl or together with the nitrogen atom to which they are attached form a 5- or 6-membered saturated ring,

or one of

$R_4$ and $R_4'$ is hydrogen and the other together with $R_3$ and the 3 carbon atoms separating them forms an aliphatic, heteroaliphatic, aromatic or heteroaromatic ring,

$R_5$ is hydroxy, acyloxy, alkoxy, benzyloxy, amino, acylamino or a group of formula

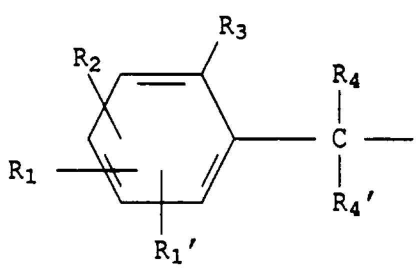

,

wherein the substituents are as defined above,

$R_6$ is carboxy, alkoxycarbonyl, benzyloxycarbonyl, phenoxycarbonyl, alkenyloxycarbonyl, $-CONR_7R_8$ wherein $R_7$ and $R_8$ are as defined above, or cyano, and

either both $R_9$ are hydrogen

or together with the carbon atoms to which they are attached form an aromatic ring,

with the proviso that at least one of $R_1$, $R_2$ and $R_3$ is other than hydrogen or chlorine,

in free form and, where such forms exist, in salt form.

Unless otherwise stated herein:

- all carbon chains may be straight or branched;
- alkyl and/or alkoxy present as or in a substituent preferably has from 1 to 12, especially 1 to 8, particularly 1 to 6 and especially 1 to 4 carbon atoms; alkyl especially is methyl; alkoxy especially is

methoxy;

- alkenyl preferably has 2 to 6, especially 2 to 4 carbon atoms; it particularly is vinyl or allyl;
- acyloxy and/or acylamino preferably is formyloxy or formylamino or alkylcarbonyloxy or alkylcarbonylamino of altogether 2 to 12, preferably 2 to 5 carbon atoms; acyloxy especially is acetoxy; acylamino especially is acetylamino;
- halogen preferably is fluorine, chlorine or bromine, especially chlorine or bromine;
- an aliphatic ring preferably is a cyclopentyl or cyclohexyl ring;
- a heteroaliphatic ring preferably has oxygen or sulfur as heteroatom; it preferably is of altogether 5 or 6 atoms; it has up to 2 heteroatoms;
- an aromatic ring preferably is phenyl;
- a heteroaromatic ring preferably is pyridyl, thienyl or furyl;
- alkoxycarbonyl preferably is of altogether 2 to 12, preferably 2 to 5 carbon atoms; it especially is methoxycarbonyl; alkenyloxycarbonyl preferably is of altogether 4 to 12, preferably 4 or 5 carbon atoms; the double bond preferably is separated from oxygen by at least 2 carbon atoms; it especially is allyloxycarbonyl.

$R_1$ and $R_1$' preferably are hydrogen or form in adjacent positions together with the carbon atoms to which they are attached an aromatic ring, e.g. phenyl, i.e. they are 1,3-butadien-1,4-diyl.

$R_2$ preferably is alkoxy or hydroxy or forms together with $R_1$ and the carbon atoms to which they are attached an aromatic ring, e.g. phenyl, or a heteroaliphatic ring, such as when $R_1$ and $R_2$ together are methylenedioxy.

$R_3$ preferably is hydroxy or alkoxy, especially alkoxy.

$R_4$ and $R_4$' preferably are hydrogen.

$R_5$ preferably is hydroxy, acetylamino or a group of formula

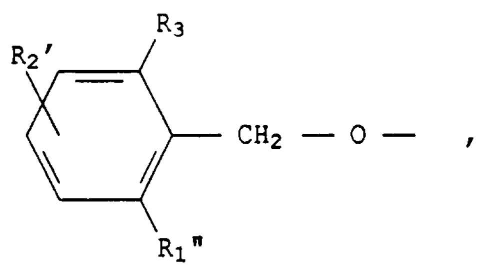

wherein

$R_1$" is hydrogen or methoxy,

$R_2$' is methoxy or acetoxy and

$R_3$ is as defined above under formula I.

$R_6$ preferably is carboxy, alkoxycarbonyl, cyano or -CONR$_7$'R$_8$' wherein either $R_7$' is hydrogen and $R_8$' is hydrogen, alkyl or benzyl or $R_7$' and $R_8$' together with the nitrogen atom to which they are attached form a 6-membered saturated ring.

Preferably either both $R_9$ are hydrogen or together with the carbon atoms to which they are attached form a phenyl ring, i.e. they are 1,3-butadien-1,4-diyl.

It will be appreciated that in formula I the benzyloxy moiety can be attached at any free position on the phenyl ring bearing $R_6$, including, when $R_9$ is hydrogen, the positions occupied in formula I by $R_9$. However, the benzyloxy moiety preferably is attached to the phenyl ring bearing $R_6$ in ortho or meta, especially in meta position, in respect to $R_6$.

In a subgroup of compounds of formula I (**compounds Io**) at least one of $R_1$, $R_2$ and $R_3$ is other than hydrogen or halogen; in a further subgroup of compounds of formula I (**compounds Ia**) $R_1$, $R_2$ and $R_3$ are other than halogen. In a further subgroup (**compounds Ib**) the benzyloxy moiety of formula I is in ortho or meta position in respect to $R_6$. In a further subgroup (**compounds Iab**) $R_1$, $R_2$ and $R_3$ are other than halogen and the benzyloxy moiety is in ortho or meta position in respect to $R_6$. In a further subgroup (**compounds Ic**) $R_4$ and $R_4$' have the significances indicated above with the exception of an aromatic or heteroaromatic ring.

In a further subgroup of compounds of formula I (**compounds Is**) either

$R_1$ is hydrogen, alkoxy of 1 to 4 carbon atoms or together with $R_2$ is methylenedioxy and

$R_1$' is hydrogen,

or

| | |
|---|---|
| $R_1$ and $R_1$' | in adjacent positions together with the carbon atoms to which they are attached form a phenyl ring, |
| $R_2$ | is hydroxy, alkoxy of 1 to 4 carbon atoms, acetoxy or together with $R_1$ is methylenedioxy, |
| $R_3$ | is hydrogen, hydroxy or alkoxy of 1 to 4 carbon atoms, |
| $R_4$ and $R_4$' | are hydrogen, |
| $R_5$ | is hydroxy, acetoxy, alkoxy of 1 to 4 carbon atoms, amino, acetylamino, 2,5-dimethoxybenzyloxy or 3-acetoxybenzyloxy, |
| $R_6$ | is carboxy, alkoxycarbonyl of altogether 2 to 5 carbon atoms, cyano, or -CONR$_7$R$_8$ wherein $R_7$ and $R_8$ either independently are hydrogen, alkyl of 1 to 4 carbon atoms or benzyl or together with the nitrogen atom to which they are attached form piperidino, |

either both $R_9$ are hydrogen

or together with the carbon atoms to which they are attached form a phenyl ring,

with the proviso that at least one of $R_1$, $R_2$ and $R_3$ is other than hydrogen.

In a subgroup of compounds Is the benzyloxy moiety is attached to the phenyl ring bearing $R_6$ in ortho or meta position in respect to $R_6$.

In a further subgroup of compounds of formula I (**compounds Ip**)

| | |
|---|---|
| $R_1$, $R_2$, $R_3$ and $R_4$ | are as defined above under formula I, |
| $R_1$' and $R_9$ | are hydrogen, |
| $R_5$ | is hydroxy, benzyloxy, alkoxy or acyloxy and |
| $R_6$ | with the exception of carboxy and cyano has the significance indicated above under formula I, |

with the proviso that at least one of $R_1$, $R_2$ and $R_3$ is other than hydrogen or chlorine.

The invention also provides a process for the preparation of the compounds of formula I in free form and, where such forms exist, in salt form which comprises reacting a compound of formula II

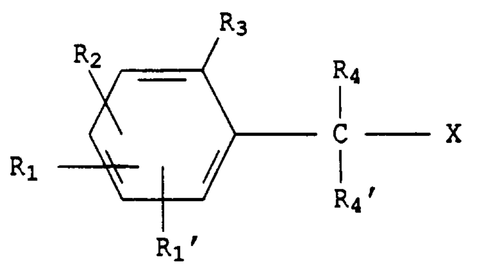

II

wherein

$R_1$, $R_1$', $R_2$, $R_3$, $R_4$ and $R_4$' are as defined above and X is a leaving group, with a compound of formula III

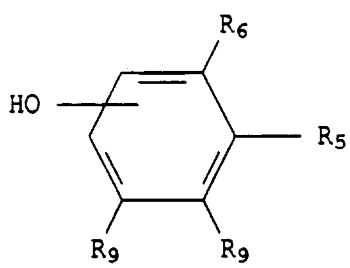

III

wherein the substituents are as defined above,

where appropriate in protected form,

and where a resultant compound is in protected form, appropriately removing the protecting group(s) therefrom, and/or converting a resultant compound of formula I into a further compound of formula I,

and recovering the compounds thus obtained in free form or, where such forms exist, in salt form.

The process of the invention is a condensation reaction. It may be carried out in conventional manner. Preferably it is effected in an inert solvent such as a carboxylic acid dialkylamide, e.g. dimethylformamide, or an oxoalkane. e.g. acetone, preferably in the presence of an acid binding agent such as an alkali metal carbonate, e.g. potassium carbonate. The reaction temperature conveniently lies between about room

temperature and the boiling point of the reaction mixture, preferably between about room temperature and 60 ° C. The leaving group conveniently is chlorine, bromine or iodine or an organic sulfonyloxy group such as an alkylsulfonyloxy group of 1 to 10 carbon atoms or an arylsulfonyloxy group, e.g. mesyloxy or tosyloxy; it preferably is chlorine or bromine, especially bromine.

In a variant the process of the invention is carried out in the presence of triphenylphosphine and an azodicarbonic acid alkyl ester such as azodicarbonic acid diethyl ester. Preferably the reaction is effected in an inert solvent such as a cyclic ether, e.g. tetrahydrofuran, preferably under an inert atmosphere, e.g. under argon. The reaction temperature conveniently lies between about room temperature and the boiling temperature of the reaction mixture, preferably at about room temperature. The leaving group is then formally hydroxy, as the O-phosphonium complex.

The compounds of formula III may comprise two hydroxy groups with different reactivities, whereby the hydroxy group adjacent to the substituent $R_6$ has the lesser reactivity. Depending on the reaction conditions employed one or both hydroxy groups may react, resulting in end products containing one or two benzyloxy groups. Preferably reaction conditions are selected which give mainly either the mono- or the disubstituted product. Separation of resultant mixtures is carried out in conventional manner, e.g. by chromatography.

Removal of protecting groups for e.g. a hydroxy moiety may also be effected in conventional manner, e.g. by hydrolysis with e.g. aqueous hydrofluoric acid solution in acetonitrile for removal of a silyl protecting group, or e.g. aqueous hydrochloric acid solution in methanol for removal of an acyl protecting group.

Conversion of a compound of formula I into a further compound of formula I may also be carried out in conventional manner. Compounds of formula I wherein $R_5$ is alkoxy, benzyloxy, acyloxy, or acylamino may e.g. be obtained according to known methods by appropriate alkylation or acylation from corresponding compounds of formula I wherein $R_5$ is hydroxy or amino. Compounds of formula I wherein $R_6$ is $-CONR_7R_8$ may e.g. be obtained by appropriate amidation from compounds of formula I wherein $R_6$ is carboxy or alkoxycarbonyl. Compounds of formula I wherein $R_6$ is alkoxycarbonyl, benzyloxycarbonyl, phenoxycarbonyl or alkenyloxycarbonyl may e.g. be obtained by appropriate esterification from compounds of formula I wherein $R_6$ is a carboxy group, and vice-versa, compounds of formula I wherein $R_6$ is carboxy may e.g. be obtained by appropriate ester group hydrolysis.

A compound of formula I in free form may be converted into a salt form where such forms exist, e.g. an acid addition salt form, in conventional manner and vice-versa.

A compound of formula I in free form or salt form may be isolated and purified from the reaction mixture in conventional manner.

The starting materials and intermediates are either known or can be prepared according to known methods.

In the following illustrative Examples all temperatures are in degrees Celsius. All NMR spectra are $^1H$-NMR spectra ($CDCl_3$ unless indicated otherwise). mp = melting point.

**Example 1: 5-(2,5-Dimethoxybenzyloxy)-2-hydroxybenzoic acid methyl ester**

[Formula I: $R_1$ = 5-$OCH_3$; $R_1'$, $R_2$, $R_4$, $R_4'$, $R_9$ = H; $R_3$ = $OCH_3$; $R_5$ = 2-OH; $R_6$ = 1-$COOCH_3$; benzyloxy moiety in 5 (meta) position in respect to $R_6$]

[Condensation]

To a mixture of 363 mg of **2,5-dihydroxybenzoic acid methyl ester** and 900 mg of potassium carbonate in 15 ml of absolute acetone are added at room temperature 500 mg of **2,5-dimethoxybenzyl bromide**. After 14 hours at 60 ° the mixture is poured onto 200 ml of water and extracted with ethyl acetate. The combined extracts are dried, evaporated and subjected to chromatography on silica gel (hexane/ethyl acetate = 5/1) to yield the **title compound** (colourless crystals; mp after recrystallization from ethanol: 110 ° );

NMR: 10.38 (s, 1H); 7.43 (d, J=3 Hz, 1H); 7.17 (dd, J=3+9Hz, 1H); 7.06 (m, 1H); 6.91 d, J=9Hz, 1H); 6.78-6.87 (m, 2H); 5.04 (s, 2H); 3.95 (s, 3H); 3.82 (s, 3H); 3.78 (s, 3H).

**Example 2: 5-(2,5-Dihydroxybenzyloxy)-2-hydroxybenzoic acid methyl ester**

[Condensation followed by deprotection]

To a solution of 0.67 g of **2,5-(tert-butyldimethylsilyloxy)benzyl alcohol**, 0.476 g of **triphenylphosphine** and 0.203 g of **2,5-dihydroxybenzoic acid methyl ester** in 5 ml of dry tetrahydrofuran is added a solution of 0.31 g of azodicarbonic acid diethylester in 5 ml of dry tetrahydrofuran within two hours and under an argon atmosphere. The reaction mixture is evaporated and subjected to silica gel chromatography (hexane/ethyl acetate = 4/1). The silyl protecting group is removed by adding 1 ml of 40 % HF in 10 ml of acetonitrile and stirring over night. The reaction mixture is then poured onto phosphate buffer (pH = 7), extracted with ethyl acetate and the combined extracts are subjected to chromatography (hexane/ethyl acetate = 4/1) to yield the **title compound** (colourless crystals; mp: 168-170°);

NMR ($CDCl_3/CD_3OD$ 1/1): 7.46 (d, J=3Hz, 1H); 7.2 (dd, J=3+9Hz, 1H); 6.9 (d, J=9Hz, 1H); 6.87 (d, J=3Hz, 1H); 6.72 (d, J=8.5Hz, 1H); 6.65 (dd, J= 3+8.5Hz, 1H); 5.02 (s, 2H); 3.95 (s, 3H).

**Example 3: 5- (2,5-Dimethoxybenzyloxy)-2-hydroxybenzoic acid**

[Conversion/deprotection by hydrolysis]

2 ml of 1 N sodium hydroxide are added at room temperature to a solution of 0,12 g of **5-(2,5-dimethoxybenzyloxy)-2-hydroxybenzoic acid methyl ester** (compound of Example 1) in 2 ml of methanol and the reaction mixture is stirred at room temperature for 48 hours. The reaction mixture is dissolved in water and washed with ethyl acetate. The aqueous phase is treated with 1 N HCl (pH = 3) and extracted with ethyl acetate. The combined extracts are evaporated to yield the **title compound** (colourless crystals; mp: 128-130°);

NMR: 7.48 (d, J=3, 1Hz, 1H); 7.17 (dd, J=3.1+9Hz, 1H); 7.05 (m, 2H); 6.90 (d, J=9Hz); 6.82 (m, 2H); 5.04 (s, 2H); 3.82 (s, 3H; 3.77 (s, 3H); 3.12 (br.s, 2H).

**Example 4: 5- (2,5-Dimethoxybenzyloxy)-2-hydroxybenzoic acid amide**

[Conversion by amidation]

The solution of 0.8 g of **5-(2,5-dimethoxybenzyloxy)-2-hydroxybenzoic acid methyl ester** - (compound of Example 1) in 10 ml of dry tetrahydrofuran is stirred at room temperature with 35 ml of saturated methanolic ammonia solution for 15 hours and then refluxed for 6 hours. The reaction mixture is evaporated and subjected to chromatography (hexane/ethyl acetate = 2/1) to yield the **title compound** - (colourless crystals; mp: 140-141°);

NMR ($d_6$-DMSO): 12.51 (br.s, 1H); 8.42 (br.s, 1H); 7.88 (br.s, 1H); 7.54 (dd, J=3Hz, 1H); 7.11 (dd, J=3+9Hz, 1H); 7.02 (d, J=3Hz, 1H); 6.97 (d, J=9Hz, 1H); 6.88 (dd, J=3+9Hz, 1H); 6.82 (d, J=9Hz, 1H); 4.98 (s, 2H); 3.77 (s, 3H); 3.71 (s, 3H).

**Example 5: 5-(2,5-Dimethoxybenzyloxy)-2-hydroxybenzonitrile**

[Conversion by dehydration]

The mixture of a solution of 0.14 g of 5-(2,5-dimethoxybenzyloxy)-2-hydroxybenzoic acid amide (compound of Example 4) in 5 ml of dry dichloromethane, 0.585 g of dry pyridine and 0.388 g of trifluoroacetic anhydride is stirred at room temperature for 15 hours. The mixture is poured onto ice water, washed twice with ethyl acetate, 1 N HCl and saturated sodium chloride solution, dried over magnesium sulfate and evaporated. The residue is subjected to chromatography (hexane/ethyl acetate = 4/1 → 2/1) to yield the **title compound** (colourless crystals; mp: 117-119°);

NMR: 7.14 (dd, J=3+9Hz, 1H); 7.06 (d, J=3Hz, 1H); 6.98-6.93 (m, 1H); 6.9 (d, J=9Hz, 1H); 6.8-6.88 (m, 2H); 5.03 (s, 2H); 3.83 (s, 3H); 3.77 (s, 3H).

**Example 6: 5-(2,5-Dimethoxybenzyloxy)-2-methoxybenzoic acid methyl ester**

[Conversion by alkylation]

The solution of 0.10 g of **5-(2,5-dimethoxybenzyloxy)-2-hydroxybenzoic acid methyl ester** -(compound of Example 1) in 2 ml of dry tetrahydrofuran is added dropwise to a suspension of 0.011 g of sodium hydride (80 % in paraffin oil) in 2 ml of dry tetrahydrofuran. After stirring for 20 minutes at room temperature 0.222 g of methyliodide in dry tetrahydrofuran are added and the mixture is stirred for 20 minutes. Then the reaction mixture is poured onto cold sodium chloride solution and extracted with ethyl acetate. The combined extracts are evaporated and subjected to chromatography (hexane/ethyl acetate = 4/1) to yield the **title compound** (oil);

NMR: 7.47 (d, J = 3Hz, 1H); 7.12 (dd, J = 3 + 9Hz, 1H); 7.03-7.09 (m, 1H); 6.92 (d, J = 9Hz, 1H); 6.77-6.88 (m, 2H); 5.06 (s, 2H); 3.9 (s, 3H); 3.87 (s, 3H); 3.83 (s, 3H); 3.78 (s, 3H).

**Example 7: 5-(2,5-Dimethoxybenzyloxy)-2-acetoxybenzoic acid methyl ester**

[Conversion by acylation]

To a solution of 0.12 g of **5-(2,5 dimethoxybenzyloxy)-2-hydroxybenzoic acid methyl ester** -(compound of Example 1) in 5 ml of dry dichloromethane 0.057 g of triethylamine, 0.05 g of acetic anhydride and 0.01 g of 4-dimethylaminopyridine are added and the mixture is stirred at room temperature for 3 hours. Then the reaction mixture is poured onto ice water, washed with saturated sodium chloride solution, dried over magnesium sulfate and evaporated. The residue is subjected to chromatography (hexane/ethyl acetate = 4/1) to yield the **title compound** (colourless crystals; mp: 72-73°);

NMR: 7.64 (d, J = 3.1Hz, 1H); 7.17 (dd, J = 3.1 + 8.8Hz, 1H); 7.03 (m, 2H); 6.85 (s, 2H); 5.10 (s, 2H); 3.87 (s, 3H); 3.83 (s, 3H); 3.79 (s, 3H); 3.78 (s, 3H); 2.33 (s, 3H).

The following compounds of formula I are obtained analogously:

| Example No. | Analogously to Ex. No. | $R_1'$ | $R_1$ | $R_2$ | $R_3$ | $R_4$, $R_4'$ | $R_5$ | $R_6$ | $R_9$ | Position[1] | Characterization |
|---|---|---|---|---|---|---|---|---|---|---|---|
| 8 | 1 | H | 3,4-$OCH_2O$- | | H | H,H | OH | $COOCH_3$ | H,H | 5 | mp 120-123°; NMR* |
| 9 | 1 | H | H | 5-$OCH_3$ | $OCH_3$ | H,H | OH | $COOCH_3$ | H,H | 4 | mp 95-97°; NMR* |
| 10 | 1 | H | H | 3-$OCH_3$ | $OCH_3$ | H,H | OH | $COOCH_3$ | H,H | 5 | mp 90-93°; NMR* |
| 11 | 1 | H | H | 3-$OCH_3$ | $OCH_3$ | H,H | OH | $COOCH_3$ | H,H | 4 | mp 93-95°; NMR* |
| 12 | 1 | H | H | 5-$OCH_3$ | $OCH_3$ | H,H | OH | $COOCH_3$ | H,H | 3 | mp 97-105°; NMR* |
| 13 | 1 | H | H | 5-$OCH_3$ | $OCH_3$ | H,H | OH | $COOCH_3$ | H,H | 6[7] | mp 95-97°; NMR* |
| 14 | 1 | H | H | 5-$OCH_3$ | $OCH_3$ | H,H | OH | $COOCH_3$ | 1,3-butadi-en-1,4-diyl | 5 | mp 127°; NMR* |
| 15 | 1 | H | H | 5-$OCH_3$ | $OCH_3$ | H,H | $NHCOCH_3$ | $COOCH_3$ | H,H | 5 | mp 116-117°; NMR* |
| 16 | -[2] | H | H | 5-$OCH_3$ | $OCH_3$ | H,H | $NH_2$ | $COOCH_3$ | H,H | 5 | mp 99°; NMR* |
| 17 | -[3] | H | H | 3-OH | H | H,H | OH | $COOCH_3$ | H,H | 5 | mp 135°; NMR* |
| 18 | 1[4] | H | H | 5-$OCH_3$ | OH | H,H | OH | $COOCH_3$ | H,H | 5 | mp 100-104°; NMR* |
| 19 | 4 | H | H | 5-$OCH_3$ | $OCH_3$ | H,H | OH | $CONHCH_2C_6H_5$ | H,H | 5 | oil; NMR* |
| 20 | 4 | H | H | 5-$OCH_3$ | $OCH_3$ | H,H | OH | 1-piperidyl-carbonyl | H,H | 5 | amorphous; NMR* |
| 21 | 4 | H | H | 5-$OCH_3$ | $OCH_3$ | H,H | OH | $CONHCH_3$ | H,H | 5 | mp 123-130°; NMR* |
| 22 | 1+6 | H | H | 5-$OCH_3$ | $OCH_3$ | H,H | 2,5-di-$OCH_3$-benzyloxy | $COOCH_3$ | H,H | 5 | mp 95-97°; NMR* |
| 23 | 1+6 | H | H | 3-$OCOCH_3$ | H | H,H | 3-acetoxy-benzyloxy | $COOCH_3$ | H,H | 5 | oil; NMR* |
| 24 | 2[6] | 4,5-(1,3-butadien-1,4-diyl)[5] | | 3-$OCH_3$[5] | $OCH_3$ | H,H | OH | $COOCH_3$ | H,H | 5 | mp 92-97°; NMR* |
| 25 | 1[8] | H | H | 3-$OCOCH_3$ | H | H,H | OH | $COOCH_3$ | H,H | 5 | [9] |
| 26 | 2[6] | H | 4-$OCH_3$ | 3-$OCH_3$ | $OCH_3$ | H,H | OH | $COOCH_3$ | H,H | 5 | mp 142-145° |

1) Position of the benzyloxy moiety in respect to $R_6$;

2) By deprotection of the compound of Example 15 with 1 N HCl in methanol;

3) By deprotection of the compound of Example 25 with 2 N HCl in methanol;

4) With deprotection analogously to Example 2;

5) i.e. the phenyl ring formed by $R_1$ and $R_1'$ is adjacent to $R_3$, and $R_2$ is in para position in respect to $R_3$;

6) Without deprotection step;

7) i.e. the benzyloxy moiety is in ortho position in respect to $R_6$;

8) Starting from **3-acetoxybenzyl bromide** (compound of formula III);

9) Deprotection gives the compound of Example 17;

*NMR: Example No.:

8: 10.38 (s, 1H); 7.39 (d, J=3Hz, 1H); 7.14 (dd, J=3+9Hz, 1H); 6.80-6.96 (m, 4H); 5.98 (s, 2H); 4.92 (s, 2H); 3.95 (s, 3H);

9: 10.95 (s, 1H); 7.72-7.78 (m, 1H); 7.0-7.03 (m, 1H); 6.8-6.88 (m, 2H); 6.56 (s, 1H); 6.51-6.57 (m, 1H); 5.11 (s, 2H); 3.9 (s, 3H); 3.82 (s, 3H); 3.76 (s, 3H);

10: 10.38 (s, 1H); 7.43 (d, J=3Hz, 1H); 7.17 (dd, J=3+9Hz, 1H); 7.03-7.12 (m, 2H); 6.87-6.95 (m, 2H); 5.07 (s, 2H); 3.96 (s, 3H); 3.89 (s, 6H);

11: 10.96 (s, 1H); 7.79 (d, J=9Hz, 1H); 7.0-7.14 (m, 2H); 6.93 (dd, J=2+8Hz, 1H); 6.48-6.6 (m, 2H); 5.12 (s, 2H); 3.92 (s, 3H); 3.89 (s, 6H);

12: 10.99 (s, 1H); 7.44 (dd, J=1.5+8Hz, 1H); 7.03-7.15 (m, 2H); 6.78-6.86 (m, 2H); 6.76 (tr, J=8Hz, 1H); 5.21 (s, 2H); 3.96 (s, 3H); 3.81 (s, 3H); 3.75 (s, 3H);

13: 11.54 (s, 1H); 7.34 (tr, J=8Hz, 1H); 7.26-7.31 (m, 1H); 6.78-6.84 (m, 2H); 6.63 (dd, J=1+8Hz, 1H); 6.55 (dd, J=1+8Hz, 1H); 5.13 (s, 2H); 4.01 (s, 3H); 3.82 (s, 3H); 3.79 (s, 3H);

14: 11.65 (s, 1H); 8.28-8.43 (m, 2H); 7.55-7.66 (m, 2H); 7.19-7.26 (m, 2H); 6.81-6.93 (m, 2H); 5.22 (s, 2H); 4.0 (s, 3H); 3.86 (s, 3H); 3.8 (s, 3H);

15: 10.78 (br.s, 1H); 8.61 (d, J=9Hz, 1H); 7.63 (d, J=3Hz, 1H); 7.21 (dd, J=3+9Hz, 1H); 7.04 (d, J=2Hz, 1H); 6.78-6.88 (m, 2H); 5.08 (s, 2H); 3.92 (s, 3H); 3.83 (s, 3H); 3.77 (s, 3H); 2.21 (s, 3H);

16: 7.5 (d, J=3Hz, 1H); 7.0-7.09 (m, 2H); 6.78-6.87 (m, 2H); 6.64 (d, J=9Hz, 1H); 5.35 (br.s, 1H); 5.02 (s, 2H); 3.86 (s, 3H); 3.82 (s, 3H); 3.78 (s, 3H); 1.6 (br.s, 1H);

17: ($CD_3OD$): 7.43 (d, J=3Hz, 1H); 7.16-7.26 (m, 2H); 6.87-6.95 (m, 3H); 6.72-6.8 (m, 1H); 4.96 (s, 2H); 3.93 (s, 3H);

18: 10.44 (s, 1H); 7.45 (d, J=3Hz, 1H); 7.17 (dd, J=3+9Hz, 1H); 6.94 (d, J=9Hz, 1H); 6.73-6.85 (m, 3H); 5.98 (br.s, 1H); 5.1 (s, 2H); 3.94 (s, 3H); 3.77 (s, 3H);

19: 11.78 (s, 1H); 7.25-7.45 (m, 5H); 7.1 (dd, J=3+9Hz, 1H); 6.98-7.04 (m, 1H); 6.89-6.98 (m, 2H); 6.75-6.84 (m, 2H); 6.51 (br.s, 1H); 5.01 (s, 2H); 4.63 (d, J=5.5Hz, 2H); 3.75 (s, 3H); 3.72 (s, 3 H);

20: 9.04 (s, 1H); 6.97-7.07 (m, 2H); 6.94 (d, J=9Hz, 1H); 6.76-6.88 (m, 3H); 5.04 (s, 2H); 3.82 (s, 3H); 3.76 (s, 3H); 3.52-3.65 (m, 4H); 1.5-1.73 (m, 6H);

21: 11.9 (s, 1H); 7.09 (dd, J=3+9Hz, 1H); 7.02-7.04 (m, 1H); 6.82-6.94 (m, 4H); 6.3 (br.s, 1H); 5.03 (s, 2H); 3.82 (s, 3H); 3.77 (s, 3H); 3.0 (d, J=5Hz, 3H);

22: 7.51 (d, J=3.1Hz, 1H); 7.31 (m, 1H); 7.08 (m, 2H); 7.00 (d, 9Hz, 1H); 6.82 (m, 4H); 5.15 (s, 2H); 5.07 (s, 2H); 3.93 (s, 3H); 3.82 (s, 3H); 3.81 (s, 3H); 3.80 (s, 3H); 3.79 (s, 3H);

23: 7.15-7.43 (m, 7H); 6.9-7.1 (m, 4H); 5.12 (s, 2H); 5.05 (s, 2H); 3.9 (s, 3H); 2.31 (s, 6H);

24: 10.39 (s, 1H); 8.24 (d, J=8.4Hz, 1H); 8.07 (d, J=8Hz, 1H); 7.45-7.57 (m, 3H); 7.2 (dd, J=3+9Hz, 1H); 6.93 (d, J=9Hz, 1H); 6.86 (s, 1H); 5.21 (s, 2H); 3.97 (s, 3H); 3.93 (s, 3H); 3.92 (s, 3H).

The compounds of Examples 2 to 7, 16, 17, 19 to 21, 24 and 26 may also be prepared in a manner analogous to Example 1.

The compounds of formula I in free form and, where salts exist, in pharmaceutically acceptable salt, e.g. pharmaceutically acceptable acid addition salt form, hereinafter briefly referred to as "the compounds of the invention", possess pharmacological activity. They are indicated for use as pharmaceuticals.

In particular, they possess antihyperproliferative/antiinflammatory and anticancer activity. The following abbreviations are used hereinafter:

| | |
|---|---|
| BSA = | bovine serum albumin |
| DMEM = | Dulbecco's modified eagle medium |
| EGF = | epidermal growth factor |
| FCS = | fetal calf serum |
| HaCaT = | the cell line known as "human adult calcium temperature" |
| KBM = | keratinocyte basal medium |
| MTT = | 3-(4,5-dimethylthiazol-2-yl)-2,5-diphenyltetrazolium bromide |
| PBS = | phosphate-buffered saline:<br>8.0 g NaCl<br>0.2 g $KH_2PO_4$<br>0.2 g KCl<br>1.15 g $Na_2HPO_4$<br>0.13 g $CaCl_2 \cdot 2H_2O$<br>0.1 g $MgCl_2 \cdot 6H_2O$<br>ad 1 l with dist. $H_2O$ |
| PBSdef. = | PBS deficient:<br>138.0 mM NaCl<br>1.5 mM KOH<br>2.7 mM KCl<br>6.5 mM $Na_2HPO_4$<br>pH 7.2 |
| RPMI-1640 = | Roswell Park Memorial Institute medium 1640 |
| SDS = | sodium dodecyl sulfate |
| TGFα = | transforming growth factor α |

Antihyperproliferative/antiinflammatory activity and/or anticancer activity in neoplastic diseases may e.g. be determined as follows:

1. Inhibition of TGFα- or EGF-specific proliferation of HACAT keratinocytes:

The spontaneously immortalized aneuploid human keratinocyte HaCaT cell line, which has a transformed phenotype in vitro but remains non-tumorigenic, is grown in DMEM H21 medium supplemented with 5 % FCS and 2.5 % glutamine. 5 ml aliquots of a cell suspension adjusted to 2 x $10^5$ cells/ml are seeded into 50 ml culture flasks and the cells are cultured for 2 to 3 days in DMEM medium supplemented with 5 % FCS. When 90-100 % confluence is attained cells are trypsinized, washed, suspended in KBM medium supplemented with 0.2 % dialyzed FCS (Gibco) and $Ca^{++}$ to a final concentration of 0.06 mM, and 2 x $10^4$ cells are seeded into 96 wells cell culture plates. Proliferation is stimulated with recombinant human TGFα or EGF (reconstituted in 0.1 M acetic acid) at various concentrations in the presence of inhibitors. Cells are grown for two days at 37° / 5 % $CO_2$. During the last 16 hours incorporation of $^3$H-thymidine (1 μCi/well) is performed. Cells are washed with ice-cold PBSdef. 3 times and trichloroacetic acid twice, solubilized in 100 μl of 0.1 N NaOH containing 1 % SDS, and radioactivity is counted. Inhibition of cell proliferation is expressed as % of the control.

In the above test the compounds of the invention exhibit $IC_{50}$-values of from about 0.05 μM to about 10 μM.

Anticancer activity may e.g. be determined as follows:

2. Inhibition of tumor cell proliferation:

Tumor cell lines, e.g. K-562, L1210, HeLa and SK-BR-3 (all available from the American Type Culture Collection, Rockville, MD 20852, USA), are grown in RPMI-1640 medium supplemented with 10 % heat-inactivated (56°C / 30 min) FCS and antibiotics (1 x GIBCO penicillin-streptomycin solution). When exponential growth for tumor cell lines growing in suspension (K-562 and L1210) or 60-90 % confluence for adherent cell lines (HeLa and SK-BR-3) is reached, cells are harvested (adherent cells are trypsinized), suspended in fresh growth medium and seeded into 96 well culture plates at concentrations ranging between 1000 and 4000 cells/well. Cells are grown for three days in a final volume of 0.2 ml/well, at 37°C in a humidified incubator equilibrated with 7 % $CO_2$, in the presence of graded concentrations of test compound. The extent of cellular proliferation is measured by a colorimetric assay using MTT (Alley et al., Cancer Res. 48 [1988] 589-601).

In the above test the compounds of the invention inhibit proliferation of the four cell lines mentioned above with $IC_{50}$ values ranging between about 0.08 $\mu$M and about 10 $\mu$M.

Antihyperproliferative/antiinflammatory activity upon topical application may e.g. be determined as follows:

3. Inhibition of oxazolone-induced allergic contact dermatitis in the mouse

Sensitization is induced by a single application of 2 % 6 oxazolone (10 $\mu$l) onto the abdominal skin of mice (8 animals per group). Hypersensitivity reaction causing pinnal swelling is elicited by a second exposure to 2 % oxazolone applied to one pinna of each animal after 8 days.

Inhibition of pinnal swelling is achieved by two topical applications of test compound (30 minutes before and after elicitation of the challenge reaction). Efficacy is determined by the difference of pinnal weights of animals treated with the drug and with the vehicle (ethanol/acetone 7:3), respectively, and expressed in % of swelling with vehicle application alone.

The compounds of the invention are therefore indicated for use as antiproliferative/antiinflammatory and anticancer agents in the treatment of hyperproliferative/inflammatory disorders and cancer such as in suppression of neoplastic diseases, e.g. inflammatory/ hyperproliferative skin diseases, skin cancer, and cutaneous and systemic manifestations of immunologically-mediated illnesses and autoimmune diseases, such as: psoriasis, atopical dermatitis, contact dermatitis and related eczematous dermatitises, seborrhoeic dermatitis, Lichen planus, Pemphigus, bullous Pemphigoid, Epidermolysis bullosa, urticaria, angioedemas, vasculitides, erythemas, cutaneous eosinophilias, Lupus erythematosus and Alopecia areata.

For this use the dosage to be used will vary, of course, depending e.g. on the particular compound employed, the mode of administration and the treatment desired. However, in general, satisfactory results are obtained when the compounds are administered at a daily dosage of from about 1 mg/kg to about 30 mg/kg animal body weight, suitably given in divided doses two to four times daily. For most large mammals the total daily dosage is from about 70 mg to about 2000 mg, conveniently administered, for example, in divided doses up to four times a day or in retard form. Unit dosage forms comprise, for example, from about 17.5 mg to about 1000 mg of the compounds in admixture with at least one solid or liquid pharmaceutically acceptable carrier or diluent.

The compounds of the invention may be administered in similar manner to known standards for use in such indications. The compounds may be admixed with conventional chemotherapeutically acceptable carriers and diluents and, optionally, further excipients, and administered e.g. orally in such forms as tablets and capsules.

The compounds may alternatively be administered topically in such conventional forms as ointments or creams, parenterally or intravenously. The concentrations of the active substance will, of course, vary depending e.g. on the compound employed, the treatment desired and the nature of the form. In general, however, satisfactory results are obtained e.g. in topical application forms at concentrations of from about 0.05 to about 5 %, particularly from about 0.1 to about 1 % by weight.

Pharmaceutical compositions comprising a compound of the invention together with at least one pharmaceutically acceptable carrier or diluent also form part of the invention.

The invention also comprises the compounds of the invention for use as pharmaceuticals, especially in the treatment of hyperproliferative/inflammatory disorders and cancer.

The compound of Example 1, i.e. 5-(2,5-dimethoxybenzyloxy)-2-hydroxybenzoic acid methyl ester, is the preferred compound as an antihyperproliferative/antiinflammatory agent. It has, for example, been determined that in the above test 1, this compound has an $IC_{50}$ of less than 1 $\mu$M, and in the above test 3. elicits 46 % inhibition of swelling at a concentration of 0.4 %, as compared with $IC_{50}$ values in test 1, of from about 5 $\mu$M to about 50 $\mu$M for genistein (Merck Index, 11th Edition, 1989, item 4281), for the tyrphostin derivative AG213 (RG50864) of formula

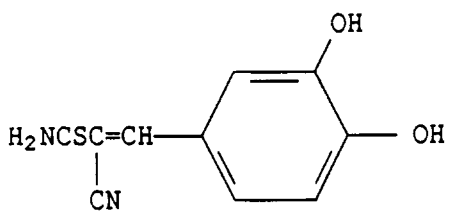

.

[A. Dvir et al., J.Cell Biol. 113 (4) (1991) 857-865] and for the partial structure of lavendustin A of formula

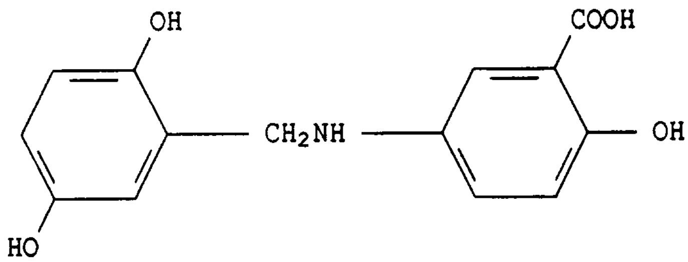

[T. Onoda et al., J.Nat.Prod. 52 (6) (1989) 1252-1257, compound 5 on page 1256], e.g. an $IC_{50}$-value of 5 $\mu$M for genistein. It is, therefore, indicated that for this use the compound of Example 1 may be administered to larger mammals, for example humans, by similar modes of administration at similar or lower dosages than conventionally employed with e.g. genistein.

The invention further includes a process for the preparation of a medicament which comprises mixing a compound of the invention with at least one pharmaceutically acceptable carrier or diluent.

## Claims

**Claims for the following Contracting States : AT, BE, CH, DE, DK, FR, GB, IT, LI, LU, NL, PT, SE**

**1.** A compound of formula I

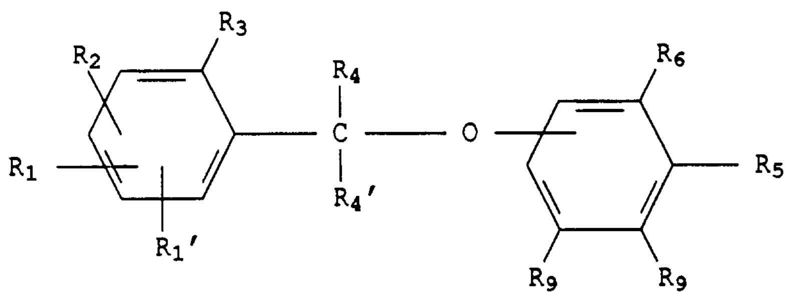

I

wherein
either

| | |
|---|---|
| $R_1$, $R_2$ and $R_3$ | independently are hydrogen, hydroxy, alkoxy, acyloxy, halogen or benzyloxy, whereby two of these substituents in adjacent positions together with the carbon atoms to which they are attached additionally can form an aliphatic, heteroaliphatic, aromatic or heteroaromatic ring and |
| $R_1'$ | is hydrogen |
| | or |
| $R_1$ and $R_1'$ | in adjacent positions together with the carbon atoms to which they are attached form an aromatic ring and |
| $R_2$ and $R_3$ | are as defined above, |
| | either |
| $R_4$ and $R_4'$ | independently are hydrogen, alkyl, alkenyl, cyano, carboxy, alkoxycarbonyl, $-CONR_7R_8$ or optionally hydroxy-, alkoxy- or halogen-substituted phenyl, whereby |
| $R_7$ and $R_8$ | either independently are hydrogen, alkyl, phenyl or benzyl or together with the nitrogen atom to which they are attached form a 5- or 6-membered saturated ring, |
| | or |
| one of $R_4$ and $R_4'$ | is hydrogen and the other together with $R_3$ and the 3 carbon atoms separating them forms an aliphatic, heteroaliphatic, aromatic or heteroaromatic ring, |
| $R_5$ | is hydroxy, acyloxy, alkoxy, benzyloxy, amino, acylamino or a group of |

formula

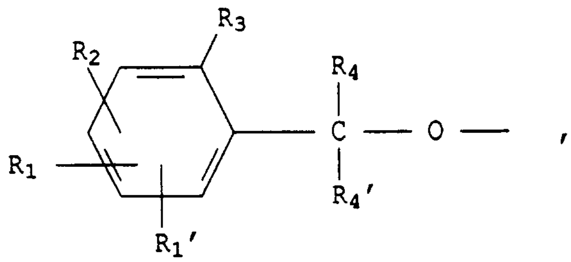

wherein the substituents are as defined above,

$R_6$ is carboxy, alkoxycarbonyl, benzyloxycarbonyl, phenoxycarbonyl, alkenyloxycarbonyl, $-CONR_7R_8$ wherein $R_7$ and $R_8$ are as defined above, or cyano, and either both $R_9$ are hydrogen

or together with the carbon atoms to which they are attached form an aromatic ring,

with the proviso that at least one of $R_1$, $R_2$ and $R_3$ is other than hydrogen or chlorine,

in free form or, where such forms exist, in salt form.

**2.** A compound according to claim 1 of formula I wherein $R_1$, $R_2$ and $R_3$ are other than halogen and the benzyloxy moiety is in ortho or meta position in respect to $R_6$, in free form or, where such forms exist, in salt form.

**3.** A compound according to claim 1 of formula I wherein
either

$R_1$ is hydrogen, alkoxy of 1 to 4 carbon atoms or together with $R_2$ is methylenedioxy and

$R_1'$ is hydrogen,

or

$R_1$ and $R_1'$ in adjacent positions together with the carbon atoms to which they are attached form a phenyl ring,

$R_2$ is hydroxy, alkoxy of 1 to 4 carbon atoms, acetoxy or together with $R_1$ is methylenedioxy,

$R_3$ is hydrogen, hydroxy or alkoxy of 1 to 4 carbon atoms,

$R_4$ and $R_4'$ are hydrogen,

$R_5$ is hydroxy, acetoxy, alkoxy of 1 to 4 carbon atoms, amino, acetylamino, 2,5-dimethoxybenzyloxy or 3-acetoxybenzyloxy,

$R_6$ is carboxy, alkoxycarbonyl of altogether 2 to 5 carbon atoms, cyano, or $-CONR_7R_8$ wherein $R_7$ and $R_8$ either independently are hydrogen, alkyl of 1 to 4 carbon atoms or benzyl or together with the nitrogen atom to which they are attached form piperidino,

either both $R_9$ are hydrogen

or together with the carbon atoms to which they are attached form a phenyl ring,

with the proviso that at least one of $R_1$, $R_2$ and $R_3$ is other than hydrogen, in free form or, where such forms exist, in salt form.

**4.** A compound according to claim 1 of formula I wherein

$R_1$, $R_2$, $R_3$ and $R_4$ are as defined in claim 1 under formula I,

$R_1'$ and $R_9$ are hydrogen,

$R_5$ is hydroxy, benzyloxy, alkoxy or acyloxy and

$R_6$ with the exception of carboxy and cyano has the significance indicated in claim 1 under formula I,

with the proviso that at least one of $R_1$, $R_2$ and $R_3$ is other than hydrogen or chlorine,

in free form or, where such forms exist, in salt form.

**5.** A compound according to claim 1 which is 5-(2,5-dimethoxybenzyloxy)-2-hydroxybenzoic acid methyl ester.

**6.** A compound according to claim 1 which is
- either 5-(2,5-dihydroxybenzyloxy)-2-hydroxybenzoic acid methyl ester
- or 5-(2,5-dimethoxybenzyloxy)-2-hydroxybenzoic acid
- or 5-(2,5-dimethoxybenzyloxy)-2-hydroxybenzoic acid amide
- or 5-(2,5-dimethoxybenzyloxy)-2-hydroxybenzonitrile
- or 5-(2,5-dimethoxybenzyloxy)-2-methoxybenzoic acid methyl ester
- or 5-(2,5-dimethoxybenzyloxy)-2-acetoxybenzoic acid methyl ester,
- or of formula I wherein $R_1'$, $R_1$, $R_4$, $R_4'$ and both $R_9$ are H and $R_2$, $R_3$, $R_5$, $R_6$ and the position of the benzyloxy moiety in respect to $R_6$ are, respectively,
  - either 5-$OCH_3$, $OCH_3$, OH, $COOCH_3$ and 4
  - or 3-$OCH_3$, $OCH_3$, OH, $COOCH_3$ and 5
  - or 3-$OCH_3$, $OCH_3$, OH, $COOCH_3$ and 4
  - or 5-$OCH_3$, $OCH_3$, OH, $COOCH_3$ and 3
  - or 5-$OCH_3$, $OCH_3$, OH, $COOCH_3$ and 6
  - or 5-$OCH_3$, $OCH_3$, $NHCOCH_3$, $COOCH_3$ and 5
  - or 5-$OCH_3$, $OCH_3$, $NH_2$, $COOCH_3$ and 5
  - or 3-OH, H, OH, $COOCH_3$ and 5
  - or 5-$OCH_3$, OH, OH, $COOCH_3$ and 5
  - or 5-$OCH_3$, $OCH_3$, OH, $CONHCH_2C_6H_5$ and 5
  - or 5-$OCH_3$, $OCH_3$, OH, 1-piperidylcarbonyl and 5
  - or 5-$OCH_3$, $OCH_3$, OH, $CONHCH_3$ and 5
  - or 5-$OCH_3$, $OCH_3$, 2,5-di-$OCH_3$-benzyloxy, $COOCH_3$ and 5
  - or 3-$OCOCH_3$, H, 3-acetoxybenzyloxy, $COOCH_3$ and 5
  - or 3-$OCOCH_3$, H, OH, $COOCH_3$ and 5,
- or of formula I wherein $R_1'$, $R_4$, $R_4'$ and both $R_9$ are H, $R_5$ is OH, $R_6$ is $COOCH_3$, the position of the benzyloxy moiety in respect to $R_6$ is 5 and $R_1$, $R_2$, $R_3$ are, respectively,
  - either 3,4-$OCH_2O$- for $R_1$ and $R_2$ and H for $R_3$
  - or 4-$OCH_3$, 3-$OCH_3$ and $OCH_3$,
- or of formula I wherein $R_3$ is $OCH_3$, $R_4$ and $R_4'$ are H, $R_5$ is OH, $R_6$ is $COOCH_3$, the position of the benzyloxy moiety in respect to $R_6$ is 5 and $R_1'$, $R_1$, $R_2$ and both $R_9$ are, respectively,
  - either H, H, 5-$OCH_3$ and 1,3-butadien-1,4-diyl
  - or 4,5-(1,3-butadien-1,4-diyl), 3-$OCH_3$, H and H,

in free form or, where such forms exist, in salt form.

**7.** A process for the preparation of a compound according to claim 1 which comprises reacting a compound of formula II

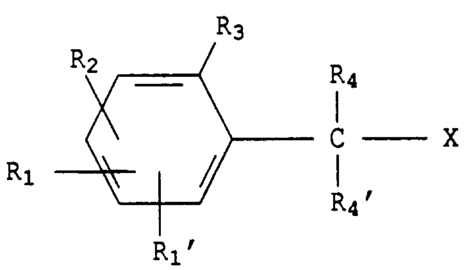

II

wherein
$R_1$, $R_1'$, $R_2$, $R_3$, $R_4$ and $R_4'$ are as defined in claim 1 and X is a leaving group,
with a compound of formula III

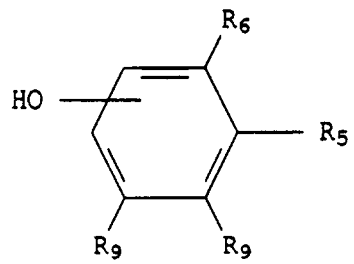

III

wherein the substituents are as defined above,
where appropriate in protected form,

and where a resultant compound is in protected form, appropriately removing the protecting group(s) therefrom, and/or converting a resultant compound of formula I into a further compound of formula I,

and recovering the compounds thus obtained in free form or, where such forms exist, in salt form.

**8.** A process according to claim 7 which is carried out in the presence of triphenylphosphine and an azodicarbonic acid alkyl ester.

**9.** A compound according to claim 1 in free form or, where salts exist, in pharmaceutically acceptable salt form for use as a pharmaceutical, especially in the treatment of hyperproliferative/inflammatory disorders and cancer.

**10.** A pharmaceutical composition comprising a compound according to claim 1 in free form or, where salts exist, in pharmaceutically acceptable salt form together with at least one pharmaceutically acceptable carrier or diluent.

**Claims for the following Contracting States : ES, GR**

**1.** A process for the preparation of a compound of formula I

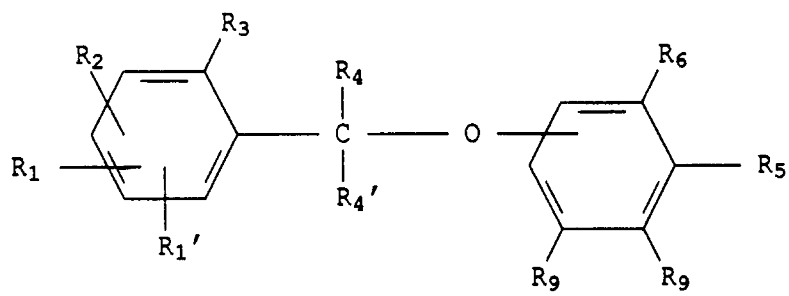

I

wherein
either

| | |
|---|---|
| $R_1$, $R_2$ and $R_3$ | independently are hydrogen, hydroxy, alkoxy, acyloxy, halogen or benzyloxy, whereby two of these substituents in adjacent positions together with the carbon atoms to which they are attached additionally can form an aliphatic, heteroaliphatic, aromatic or heteroaromatic ring and |
| $R_1'$ | is hydrogen |
| | or |
| $R_1$ and $R_1'$ | in adjacent positions together with the carbon atoms to which they are attached form an aromatic ring and |
| $R_2$ and $R_3$ | are as defined above, |
| | either |
| $R_4$ and $R_4'$ | independently are hydrogen, alkyl, alkenyl, cyano, carboxy, alkoxycarbonyl, |

-$CONR_7R_8$ or optionally hydroxy-, alkoxy- or halogen-substituted phenyl, whereby

$R_7$ and $R_8$ either independently are hydrogen, alkyl, phenyl or benzyl or together with the nitrogen atom to which they are attached form a 5- or 6-membered saturated ring,

or

one of $R_4$ and $R_4$' is hydrogen and the other together with $R_3$ and the 3 carbon atoms separating them forms an aliphatic, heteroaliphatic, aromatic or heteroaromatic ring,

$R_5$ is hydroxy, acyloxy, alkoxy, benzyloxy, amino, acylamino or a group of formula

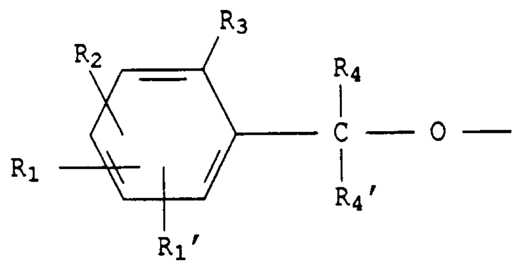

,

wherein the substituents are as defined above,

$R_6$ is carboxy, alkoxycarbonyl, benzyloxycarbonyl, phenoxycarbonyl, alkenyloxycarbonyl, -$CONR_7R_8$ wherein $R_7$ and $R_8$ are as defined above, or cyano, and

either both $R_9$ are hydrogen

or together with the carbon atoms to which they are attached form an aromatic ring,

with the proviso that at least one of $R_1$, $R_2$ and $R_3$ is other than hydrogen or chlorine,

in free form and, where such forms exist, in salt form,

which comprises reacting a compound of formula II

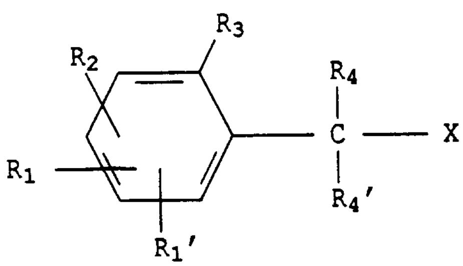

II

wherein

$R_1$, $R_1$', $R_2$, $R_3$, $R_4$ and $R_4$' are as defined in this claim and X is a leaving group,

with a compound of formula III

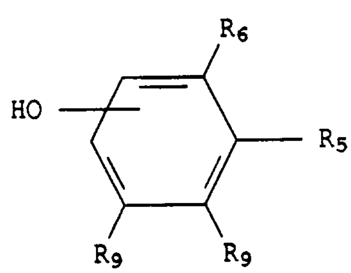

III

wherein the substituents are as defined in this claim, where appropriate in protected form,

and where a resultant compound is in protected form, appropriately removing the protecting group(s) therefrom, and/or converting a resultant compound of formula I into a further compound of formula I,

and recovering the compounds thus obtained in free form or, where such forms exist, in salt form.

**2.** A process according to claim 1 which is carried out in the presence of triphenylphosphine and an azodicarbonic acid alkyl ester.

**3.** A process according to claim 1 or 2 for the preparation of a compound according to claim 1 of formula I wherein $R_1$, $R_2$ and $R_3$ are other than halogen and the benzyloxy moiety is in ortho or meta position in respect to $R_6$, in free form or, where such forms exist, in salt form.

**4.** A process according to claim 1 or 2 for the preparation of a compound according to claim 1 of formula I wherein

either

| | |
|---|---|
| $R_1$ | is hydrogen, alkoxy of 1 to 4 carbon atoms or together with $R_2$ is methylenedioxy and |
| $R_1'$ | is hydrogen, |
| | or |
| $R_1$ and $R_1'$ | in adjacent positions together with the carbon atoms to which they are attached form a phenyl ring, |
| $R_2$ | is hydroxy, alkoxy of 1 to 4 carbon atoms, acetoxy or together with $R_1$ is methylenedioxy, |
| $R_3$ | is hydrogen, hydroxy or alkoxy of 1 to 4 carbon atoms, |
| $R_4$ and $R_4'$ | are hydrogen, |
| $R_5$ | is hydroxy, acetoxy, alkoxy of 1 to 4 carbon atoms, amino, acetylamino, 2,5-dimethoxybenzyloxy or 3-acetoxybenzyloxy, |
| $R_6$ | is carboxy, alkoxycarbonyl of altogether 2 to 5 carbon atoms, cyano, or $-CONR_7R_8$ wherein $R_7$ and $R_8$ either independently are hydrogen, alkyl of 1 to 4 carbon atoms or benzyl or together with the nitrogen atom to which they are attached form piperidino, either both $R_9$ are hydrogen |

or together with the carbon atoms to which they are attached form a phenyl ring,

with the proviso that at least one of $R_1$, $R_2$ and $R_3$ is other than hydrogen,

in free form or, where such forms exist, in salt form.

**5.** A process according to claim 1 or 2 for the preparation of a compound according to claim 1 of formula I wherein

| | |
|---|---|
| $R_1$, $R_2$, $R_3$ and $R_4$ | are as defined in claim 1 under formula I, |
| $R_1'$ and $R_9$ | are hydrogen, |
| $R_5$ | is hydroxy, benzyloxy, alkoxy or acyloxy and |
| $R_6$ | with the exception of carboxy and cyano has the significance indicated in claim 1 under formula I, |

with the proviso that at least one of $R_1$, $R_2$ and $R_3$ is other than hydrogen or chlorine,

in free form or, where such forms exist, in salt form.

**6.** A process according to claim 1 or 2 for the preparation of 5-(2,5-dimethoxybenzyloxy)-2-hydroxybenzoic acid methyl ester.

**7.** A process according to claim 1 or 2 for the preparation of a compound according to claim 1 which is

- either 5-(2,5-dihydroxybenzyloxy)-2-hydroxybenzoic acid methyl ester
- or 5-(2,5-dimethoxybenzyloxy)-2-hydroxybenzoic acid
- or 5-(2,5-dimethoxybenzyloxy)-2-hydroxybenzoic acid amide
- or 5-(2,5-dimethoxybenzyloxy)-2-hydroxybenzonitrile
- or 5-(2,5-dimethoxybenzyloxy)-2-methoxybenzoic acid methyl ester

- or 5-(2,5-dimethoxybenzyloxy)-2-acetoxybenzoic acid methyl ester,
- or of formula I wherein $R_1$', $R_1$, $R_4$, $R_4$' and both $R_9$ are H and $R_2$, $R_3$, $R_5$, $R_6$ and the position of the benzyloxy moiety in respect to $R_6$ are, respectively,
  - either 5-$OCH_3$, $OCH_3$, OH, $COOCH_3$ and 4
  - or 3-$OCH_3$, $OCH_3$, OH, $COOCH_3$ and 5
  - or 3-$OCH_3$, $OCH_3$, OH, $COOCH_3$ and 4
  - or 5-$OCH_3$, $OCH_3$, OH, $COOCH_3$ and 3
  - or 5-$OCH_3$, $OCH_3$, OH, $COOCH_3$ and 6
  - or 5-$OCH_3$, $OCH_3$, $NHCOCH_3$, $COOCH_3$ and 5
  - or 5-$OCH_3$, $OCH_3$, $NH_2$, $COOCH_3$ and 5
  - or 3-OH, H, OH, $COOCH_3$ and 5
  - or 5-$OCH_3$, OH, OH, $COOCH_3$ and 5
  - or 5-$OCH_3$, $OCH_3$, OH, $CONHCH_2C_6H_5$ and 5
  - or 5-$OCH_3$, $OCH_3$, OH, 1-piperidylcarbonyl and 5
  - or 5-$OCH_3$, $OCH_3$, OH, $CONHCH_3$ and 5
  - or 5-$OCH_3$, $OCH_3$, 2,5-di-$OCH_3$-benzyloxy, $COOCH_3$ and 5
  - or 3-$OCOCH_3$, H, 3-acetoxybenzyloxy, $COOCH_3$ and 5
  - or 3-$OCOCH_3$, H, OH, $COOCH_3$ and 5,
- or of formula I wherein $R_1$', $R_4$, $R_4$' and both $R_9$ are H, $R_5$ is OH, $R_6$ is $COOCH_3$, the position of the benzyloxy moiety in respect to $R_6$ is 5 and $R_1$, $R_2$, $R_3$ are, respectively,
  - either 3,4-$OCH_2O$- for $R_1$ and $R_2$ and H for $R_3$
  - or 4-$OCH_3$, 3-$OCH_3$ and $OCH_3$,
- or of formula I wherein $R_3$ is $OCH_3$, $R_4$ and $R_4$' are H, $R_5$ is OH, $R_6$ is $COOCH_3$, the position of the benzyloxy moiety in respect to $R_6$ is 5 and $R_1$', $R_1$, $R_2$ and both $R_9$ are, respectively,
  - either H, H, 5-$OCH_3$ and 1,3-butadien-1,4-diyl
  - or 4,5-(1,3-butadien-1,4-diyl), 3-$OCH_3$, H and H,

in free form or, where such forms exist, in salt form.

8. A process for the preparation of a medicament which comprises mixing a compound of formula I as defined in claim 1 in free form or, where salts exist, in pharmaceutically acceptable salt form with at least one pharmaceutically acceptable carrier or diluent.

**Patentansprüche**
**Patentansprüche für folgende Vertragsstaaten : AT, BE, CH, DE, DK, FR, GB, IT, LI, LU, NL, PT, SE**

1. Verbindung der Formel I

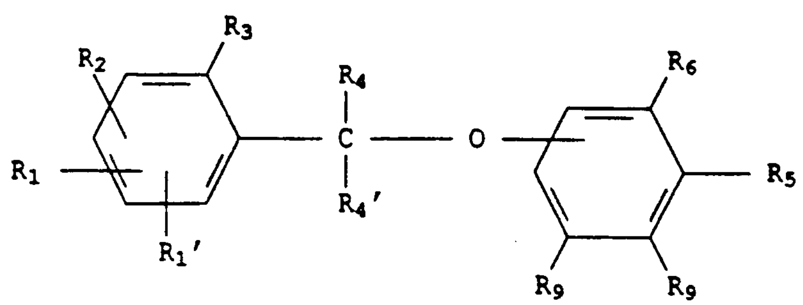

I

worin
entweder
$R_1$, $R_2$ und $R_3$ unabhängig Wasserstoff, Hydroxy-, Alkoxy-, Acyloxy-, Halogen- oder Benzyloxyreste sind, wobei zwei dieser Substituenten in benachbarten Positionen zueinander mit den Kohlenstoffatomen, an die sie gebunden sind, zusätzlich einen aliphatischen, heteroaliphatischen, aromatischen oder heteroaromatischen Ring bilden können und
$R_1$' Wasserstoff ist
oder
$R_1$ und $R_1$' in benachbarten Positionen zusammen mit den Kohlenstoffatomen, an die sie gebunden

sind, einen aromatischen Ring bilden und

$R_2$ und $R_3$ wie oben definiert sind,

entweder

$R_4$ und $R_4$' unabhängig Wasserstoff, Alkyl-, Alkenyl-, Cyano-, Carboxy-, Alkoxycarbonyl-, -CONR$_7$R$_8$- oder gegebenenfalls mit Hydroxy-, Alkoxy- oder Halogenresten substituierte Phenylreste sind, wobei $R_7$ und $R_8$ entweder unabhängig Wasserstoff, Alkyl-, Phenyl- oder Benzylreste sind oder zusammen mit dem Stickstoffatom, an das sie gebunden sind, einen 5- oder 6-gliedrigen gesättigten Ring bilden,

oder

einer der Reste $R_4$ und $R_4$' Wasserstoff ist und der andere zusammen mit $R_3$ und den 3 Kohlenstoffatomen, die sie voneinander trennen, einen aliphatischen, heteroaliphatischen, aromatischen oder heteroaromatischen Ring bildet,

$R_5$ ein Hydroxy-, Acyloxy-, Alkoxy-, Benzyloxy-, Amino-, Acylaminorest oder eine Gruppe der Formel

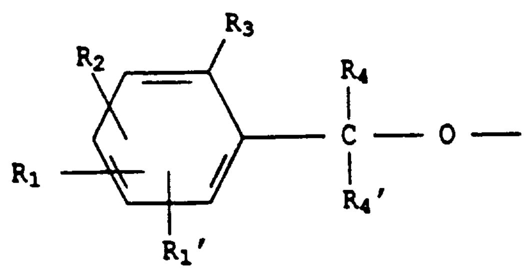

ist, worin die Substituenten wie oben definiert sind,

$R_6$ ein Carboxy-, Alkoxycarbonyl-, Benzyloxycarbonyl-, Phenoxycarbonyl-, Alkenyloxycarbonylrest, -CONR$_7$R$_8$, worin $R_7$ und $R_8$ wie oben definiert sind, oder ein Cyanorest ist und

entweder beide Reste $R_9$ Wasserstoff sind

oder zusammen mit den Kohlenstoffatomen, an die sie gebunden sind, einen aromatischen Ring bilden,

mit dem Vorbehalt, daß mindestens einer der Reste $R_1$, $R_2$ und $R_3$ etwas anderes als Wasserstoff oder Chlor bedeutet, in freier Form und, falls solche Formen existieren, in Salzform.

2. Verbindung nach Anspruch 1 der Formel I, worin $R_1$, $R_2$ und $R_3$ etwas anderes als Halogen bedeuten und der Benzyloxyrest in ortho- oder meta-Stellung bezüglich $R_6$ ist, in freier Form oder, wenn diese Formen existieren, in Salzform.

3. Verbindung nach Anspruch 1 der Formel I, worin entweder

$R_1$ Wasserstoff, ein Alkoxyrest mit 1 bis 4 Kohlenstoffatomen ist oder zusammen mit $R_2$ einen Methylendioxyrest bildet und

$R_1$'Wasserstoff ist,

oder

$R_1$ und $R_1$' in benachbarten Positionen zusammen mit den Kohlenstoffatomen, an die sie gebunden sind, einen Phenylring bilden,

$R_2$ ein Hydroxyrest, ein Alkoxyrest mit 1 bis 4 Kohlenstoffatomen, ein Acetoxyrest ist oder zusammen mit $R_1$ einen Methylendioxyrest bildet,

$R_3$ Wasserstoff, ein Hydroxyrest oder ein Alkoxyrest mit 1 bis 4 Kohlenstoffatomen ist,

$R_4$ und $R_4$' Wasserstoff sind,

$R_5$ ein Hydroxy-, Acetoxy-, Alkoxyrest mit 1 bis 4 Kohlenstoffatomen, Amino-, Acetylamino-, 2,5-Dimethoxybenzyloxy- oder 3-Acetoxybenzyloxyrest ist,

$R_6$ ein Carboxyrest, ein Alkoxycarbonylrest mit insgesamt 2 bis 5 Kohlenstoffatomen, ein Cyanorest oder -CONR$_7$R$_8$ ist, worin $R_7$ und $R_8$ entweder unabhängig Wasserstoff, Alkylreste mit 1 bis 4 Kohlenstoffatomen oder Benzylreste sind, oder zusammen mit dem Stickstoffatom, an das sie gebunden sind, einen Piperidinorest bilden,

entweder beide Reste $R_9$ Wasserstoff sind

oder zusammen mit den Kohlenstoffatomen, an die sie gebunden sind, einen Phenylring

bilden,
mit dem Vorbehalt, daß mindestens einer der Reste $R_1$, $R_2$ und $R_3$ etwas anderes als Wasserstoff ist, in freier Form oder, wenn diese Formen existieren, in Salzform.

**4.** Verbindung nach Anspruch 1 der Formel I, worin
$R_1$, $R_2$, $R_3$ und $R_4$ wie in Anspruch 1 bei Formel I definiert sind,
$R_1'$ und $R_9$ Wasserstoff sind,
$R_5$ ein Hydroxy-, Benzyloxy, Alkoxy- oder Acyloxyrest ist und
$R_6$ mit Ausnahme eines Carboxy- und Cyanorestes die in Anspruch 1 für Formel I angegebene Bedeutung hat,
mit dem Vorbehalt, daß mindestens einer der Reste $R_1$, $R_2$ und $R_3$ etwas anderes als Wasserstoff oder Chlor ist,
in freier Form oder, wenn diese Formen existieren, in Salzform.

**5.** Verbindung nach Anspruch 1, die 5-(2,5-Dimethoxybenzyloxy)-2-hydroxybenzoesäuremethylester ist.

**6.** Verbindung nach Anspruch 1, die

- entweder 5-(2,5-Dihydroxybenzyloxy)-2-hydroxybenzosäuremethylester
- oder 5-(2,5-Dimethoxybenzyloxy)-2-hydroxybenzoesäure
- oder 5-(2,5-Dimethoxybenzyloxy)-2-hydroxybenzoesäureamid
- oder 5-(2,5-Dimethoxybenzyloxy)-2-hydroxybenzonitril
- oder 5-(2,5-Dimethoxybenzyloxy)-2-methoxybenzoesäuremethylester,
- oder 5-(2,5-Dimethoxybenzyloxy)-2-acetoxybenzoesäuremethylester ist,
- oder eine Verbindung der Formel I ist, worin $R_1'$, $R_1$, $R_4$, $R_4'$ und beide Reste $R_9$ H sind und $R_2$, $R_3$, $R_5$, $R_6$ bzw. die Position des Benzyloxyrestes bezüglich $R_6$ jeweils ist:
- entweder 5-$OCH_3$, $OCH_3$, OH, $COOCH_3$ und 4
- oder 3-$OCH_3$, $OCH_3$, OH, $COOCH_3$ und 5
- oder 3-$OCH_3$, $OCH_3$, OH, $COOCH_3$ und 4
- oder 5-$OCH_3$, $OCH_3$, OH, $COOCH_3$ und 3
- oder 5-$OCH_3$, $OCH_3$, OH, $COOCH_3$ und 6
- oder 5-$OCH_3$, $OCH_3$, $NHCOCH_3$, $COOCH_3$ und 5
- oder 5-$OCH_3$, $OCH_3$, $NH_2$, $COOCH_3$ und 5
- oder 3-OH, H, OH, $COOCH_3$ und 5
- oder 5-$OCH_3$, OH, OH, $COOCH_3$ und 5
- oder 5-$OCH_3$, $OCH_3$, OH, $CONHCH_2C_6H_5$ und 5
- oder 5-$OCH_3$, $OCH_3$, OH, 1-Piperidylcarbonyl und 5
- oder 5-$OCH_3$, $OCH_3$, OH, $CONHCH_3$ und 5
- oder 5-$OCH_3$, $OCH_3$, 2,5-Di-$OCH_3$-benzyloxy, $COOCH_3$ und 5
- oder 3-$OCOCH_3$, H, 3-Acetoxybenzyloxy, $COOCH_3$ und 5
- oder 3-$OCOCH_3$, H, OH, $COOCH_3$ und 5,
- oder die Verbindung der Formel I ist, worin $R_1'$, $R_4$, $R_4'$ und beide Reste $R_9$ H sind, $R_5$ OH ist, $R_6$ $COOCH_3$ ist, die Position des Benzyloxyrestes bezüglich $R_6$ 5 ist, und $R_1$, $R_2$, $R_3$
- entweder 3,4-$OCH_2O$- für $R_1$ und $R_2$ und H für $R_3$
- oder 4-$OCH_3$, 3-$OCH_3$ und $OCH_3$ sind,
- oder die Verbindung der Formel I ist, worin $R_3$ $OCH_3$ ist, $R_4$ und $R_4'$ H sind, $R_5$ OH ist, $R_6$ $COOCH_3$ ist, die Position des Benzyloxyrestes bezüglich $R_6$ 5 ist, und $R_1'$, $R_1$, $R_2$ und beide Reste $R_9$
- entweder H, H, 5-$OCH_3$ und 1,3-Butadien-1,4-diyl
- oder 4,5-(1,3-Butadien-1,4-diyl), 3-$OCH_3$, H und H sind,

in freier Form oder, wenn diese Formen existieren, in Salzform.

**7.** Verfahren zur Herstellung einer Verbindung nach Anspruch 1, das umfaßt, daß man eine Verbindung der Formel II

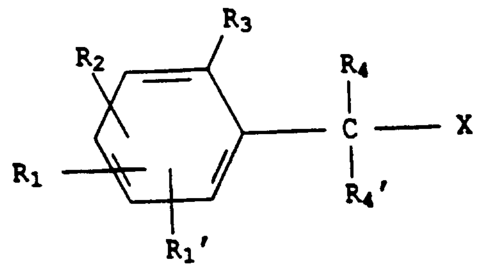

II

worin $R_1$, $R_1'$, $R_2$, $R_3$, $R_4$ und $R_4'$ wie in Anspruch 1 definiert sind und X eine Abgangsgruppe ist, mit einer Verbindung der Formel III

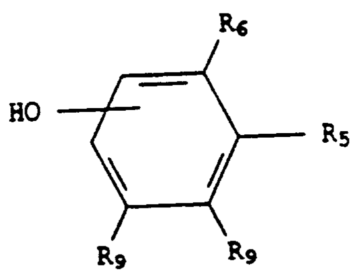

III

worin die Substituenten wie oben definiert sind, je nachdem in geschützter Form, umsetzt und, wenn eine entstehende Verbindung in geschützter Form ist, in geeigneter Weise die Schutzgruppe(n) entfernt und/oder eine entstehende Verbindung der Formel I in eine andere Verbindung der Formel I umwandelt und die so erhaltenen Verbindungen in freier Form oder, wenn diese Formen existieren, in Salzform gewinnt.

**8.** Verfahren nach Anspruch 7, das in Gegenwart von Triphenylphosphin und einem Azodicarbonsäurealkylester durchgeführt wird.

**9.** Verbindung nach Anspruch 1 in freier Form oder, wenn diese Salze existieren, in Form eines pharmazeutisch annehmbaren Salzes zur Verwendung als pharmazeutisches Mittel, insbesondere zur Behandlung von hyperproliferativen/entzündlichen Störungen und Krebs.

**10.** Pharmazeutische Zusammensetzung, umfassend eine Verbindung nach Anspruch 1 in freier Form oder, wenn diese Salze existieren, in Form des pharmazeutisch annehmbaren Salzes zusammen mit mindestens einem pharmazeutisch annehmbaren Träger oder Verdünnungsmittel.

**Patentansprüche für folgende Vertragsstaaten : ES, GR**

**1.** Verfahren zur Herstellung einer Verbindung der Formel I

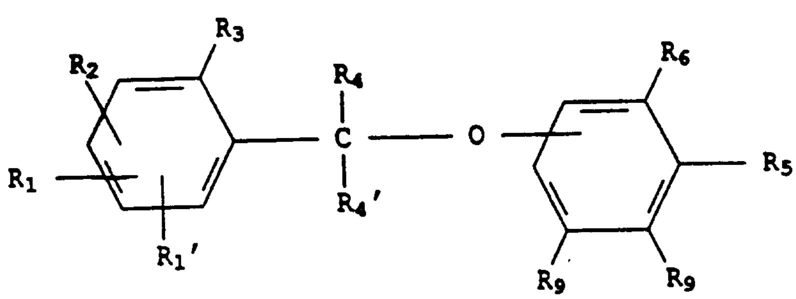

I

worin
entweder

$R_1$, $R_2$ und $R_3$ unabhängig Wasserstoff, Hydroxy-, Alkoxy-, Acyloxy-, Halogen- oder Benzyloxyreste sind, wobei zwei dieser Substituenten in benachbaren Positionen zueinander mit den Kohlenstoffatomen, an die sie gebunden sind, zusätzlich einen aliphatischen, heteroaliphatischen, aromatischen oder heteroaromatischen Ring bilden können und

$R_1$' Wasserstoff ist

oder

$R_1$ und $R_1$' in benachbarten Positionen zusammen mit den Kohlenstoffatomen, an die sie gebunden sind, einen aromatischen Ring bilden und

$R_2$ und $R_3$ wie oben definiert sind,

entweder

$R_4$ und $R_4$' unabhängig Wasserstoff, Alkyl-, Alkenyl-, Cyano-, Carboxy-, Alkoxycarbonyl-, -$CONR_7R_8$- oder gegebenenfalls mit Hydroxy-, Alkoxy- oder Halogenresten substituierte Phenylreste sind, wobei $R_7$ und $R_8$ entweder unabhängig Wasserstoff, Alkyl-, Phenyl- oder Benzylreste sind oder zusammen mit dem Stickstoffatom, an das sie gebunden sind, einen 5- oder 6-gliedrigen gesättigten Ring bilden,

oder

einer der Reste $R_4$ und $R_4$' Wasserstoff ist und der andere zusammen mit $R_3$ und den 3 Kohlenstoffatomen, die sie voneinander trennen, einen aliphatischen, heteroaliphatischen, aromatischen oder heteroaromatischen Ring bildet,

$R_5$ ein Hydroxy-, Acyloxy-, Alkoxy-, Benzyloxy-, Amino-, Acylaminorest oder eine Gruppe der Formel

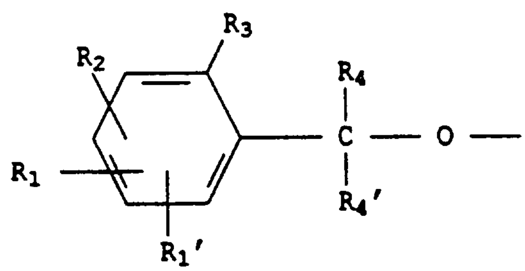

ist, worin die Substituenten wie oben definiert sind,

$R_6$ ein Carboxy-, Alkoxycarbonyl-, Benzyloxycarbonyl-, Phenoxycarbonyl-, Alkenyloxycarbonylrest, -$CONR_7R_8$, worin $R_7$ und $R_8$ wie oben definiert sind, oder ein Cyanorest ist und

entweder beide Reste $R_9$ Wasserstoff sind

oder- zusammen mit den Kohlenstoffatomen, an die sie gebunden sind, einen aromatischen Ring bilden,

mit dem Vorbehalt, daß mindestens einer der Reste $R_1$, $R_2$ und $R_3$ etwas anderes als Wasserstoff oder Chlor bedeutet, in freier Form und, falls solche Formen existieren, in Salzform, das umfaßt, daß man eine Verbindung der Formel II

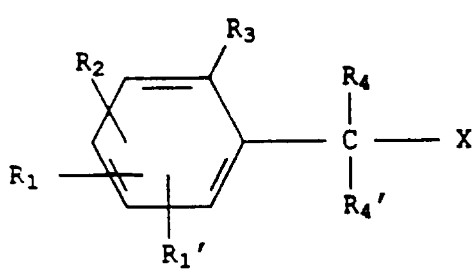

II

worin $R_1$, $R_1$', $R_2$, $R_3$, $R_4$ und $R_4$' wie in diesem Anspruch definiert sind und X eine Abgangsgruppe ist, mit einer Verbindung der Formel III

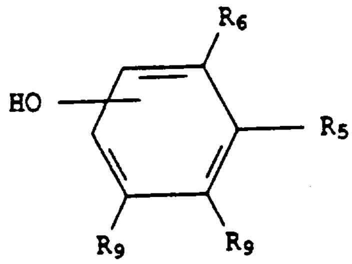

III

worin die Substituenten wie in diesem Anspruch definiert sind, je nachdem in geschützter Form, umsetzt und, wenn eine entstehende Verbindung in geschützter Form ist, in geeigneter Weise die Schutzgruppe(n) entfernt und/oder eine entstehende Verbindung der Formel I in eine andere Verbindung der Formel I umwandelt und die so erhaltenen Verbindungen in freier Form oder, wenn diese Formen existieren, in Salzform gewinnt.

**2.** Verfahren nach Anspruch 1, das in Gegenwart von Triphenylphosphin und einem Azodicarbonsäurealkylester durchgeführt wird.

**3.** Verfahren nach Anspruch 1 oder 2 zur Herstellung einer Verbindung nach Anspruch 1 der Formel I, worin $R_1$, $R_2$ und $R_3$ etwas anderes als Halogen bedeuten und der Benzyloxyrest in ortho- oder meta-Stellung bezüglich $R_6$ ist, in freier Form oder, wenn diese Formen existieren, in Salzform.

**4.** Verfahren nach Anspruch 1 oder 2 zur Herstellung einer Verbindung nach Anspruch 1 der Formel I, worin

entweder

$R_1$ Wasserstoff, ein Alkoxyrest mit 1 bis 4 Kohlenstoffatomen ist oder zusammen mit $R_2$ einen Methylendioxyrest bildet und

$R_1$'Wasserstoff ist,

oder

$R_1$ und $R_1$' in benachbarten Positionen zusammen mit den Kohlenstoffatomen, an die sie gebunden sind, einen Phenylring bilden,

| | |
|---|---|
| $R_2$ | ein Hydroxyrest, ein Alkoxyrest mit 1 bis 4 Kohlenstoffatomen, ein Acetoxyrest ist oder zusammen mit $R_1$ einen Methylendioxyrest bildet, |
| $R_3$ | Wasserstoff, ein Hydroxyrest oder ein Alkoxyrest mit 1 bis 4 Kohlenstoffatomen ist, |
| $R_4$ und $R_4$' | Wasserstoff sind, |
| $R_5$ | ein Hydroxy-, Acetoxy-, Alkoxyrest mit 1 bis 4 Kohlenstoffatomen, Amino-, Acetylamino-, 2,5-Dimethoxybenzyloxy- oder 3-Acetoxybenzyloxyrest ist, |
| $R_6$ | ein Carboxyrest, ein Alkoxycarbonylrest mit insgesamt 2 bis 5 Kohlenstoffatomen, ein Cyanorest oder $-CONR_7R_8$ ist, worin $R_7$ und $R_8$ entweder unabhängig Wasserstoff, Alkylreste mit 1 bis 4 Kohlenstoffatomen oder Benzylreste sind, oder zusammen mit dem Stickstoffatom, an das sie gebunden sind, einen Piperidinorest bilden, |
| entweder | beide Reste $R_9$ Wasserstoff sind |
| oder | zusammen mit den Kohlenstoffatomen, an die sie gebunden sind, einen Phenylring bilden, |

mit dem Vorbehalt, daß mindestens einer der Reste $R_1$, $R_2$ und $R_3$ etwas anderes als Wasserstoff ist, in freier Form oder, wenn diese Formen existieren, in Salzform.

**5.** Verfahren nach Anspruch 1 oder 2 zur Herstellung einer Verbindung nach Anspruch 1 der Formel I, worin

$R_1$, $R_2$, $R_3$ und $R_4$ wie in Anspruch 1 bei Formel I definiert sind,

$R_1$' und $R_9$ Wasserstoff sind,

$R_5$ ein Hydroxy-, Benzyloxy-, Alkoxy- oder Acyloxyrest ist und

$R_6$ mit Ausnahme eines Carboxy- und Cyanorestes die in Anspruch 1 für Formel I angegebene Bedeutung hat,

mit dem Vorbehalt, daß mindestens einer der Reste $R_1$, $R_2$ und $R_3$ etwa anderes als Wasserstoff oder Chlor ist,

in freier Form oder, wenn diese Formen existieren, in Salzform.

**6.** Verfahren nach Anspruch 1 oder 2 zur Herstellung von 5-(2,5-Dimethoxybenzyloxy)-2-hydroxybenzoesäuremethylester.

**7.** Verfahren nach Anspruch 1 oder 2 zur Herstellung einer Verbindung nach Anspruch 1, die

- entweder 5-(2,5-Dihydroxybenzyloxy)-2-hydroxybenzoesäuremethylester
- oder 5-(2,5-Dimethoxybenzyloxy)-2-hydroxybenzoesäure
- oder 5-(2,5-Dimethoxybenzyloxy)-2-hydroxybenzoesäureamid
- oder 5-(2,5-Dimethoxybenzyloxy)-2-hydroxybenzonitril
- oder 5-(2,5-Dimethoxybenzyloxy)-2-methoxybenzoesäuremethylester
- oder 5-(2,5-Dimethoxybenzyloxy)-2-acetoxybenzoesäuremethylester ist,
- oder eine Verbindung der Formel I ist, worin $R_1'$, $R_1$, $R_4$, $R_4'$ und beide Reste $R_9$ H sind und $R_2$, $R_3$, $R_5$, $R_6$ bzw. die Position des Benzyloxyrestes bezüglich $R_6$ jeweils ist:
- entweder 5-$OCH_3$, $OCH_3$, OH, $COOCH_3$ und 4
- oder 3-$OCH_3$, $OCH_3$, OH, $COOCH_3$ und 5
- oder 3-$OCH_3$, $OCH_3$, OH, $COOCH_3$ und 4
- oder 5-$OCH_3$, $OCH_3$, OH, $COOCH_3$ und 3
- oder 5-$OCH_3$, $OCH_3$, OH, $COOCH_3$ und 6
- oder 5-$OCH_3$, $OCH_3$, $NHCOCH_3$, $COOCH_3$ und 5
- oder 5-$OCH_3$, $OCH_3$, $NH_2$, $COOCH_3$ und 5
- oder 3-OH, H, OH, $COOCH_3$ und 5
- oder 5-$OCH_3$, OH, OH, $COOCH_3$ und 5
- oder 5-$OCH_3$, $OCH_3$, OH, $CONHCH_2C_6H_5$ und 5
- oder 5-$OCH_3$, $OCH_3$, OH, 1-Piperidylcarbonyl und 5
- oder 5-$OCH_3$, $OCH_3$, OH, $CONHCH_3$ und 5
- oder 5-$OCH_3$, $OCH_3$, 2,5-Di-$OCH_3$-benzyloxy, $COOCH_3$ und 5
- oder 3-$OCOCH_3$, H, 3-Acetoxybenzyloxy, $COOCH_3$ und 5
- oder 3-$OCOCH_3$, H, OH, $COOCH_3$ und 5,
- oder die Verbindung der Formel I ist, worin $R_1'$, $R_4$, $R_4'$ und beide Reste $R_9$ H sind, $R_5$ OH ist, $R_6$ $COOCH_3$ ist, die Position des Benzyloxyrestes bezüglich $R_6$ 5 ist, und $R_1$, $R_2$, $R_3$
- entweder 3,4-$OCH_2O$- für $R_1$ und $R_2$ und H für $R_3$
- oder 4-$OCH_3$, 3-$OCH_3$ und $OCH_3$ sind,
- oder die Verbindung der Formel I ist, worin $R_3$ $OCH_3$ ist, $R_4$ und $R_4'$ H sind, $R_5$ OH ist, $R_6$ $COOCH_3$ ist, die Position des Benzyloxyrestes bezüglich $R_6$ 5 ist, und $R_1'$, $R_1$, $R_2$ und beide Reste $R_9$
- entweder H, H, 5-$OCH_3$ und 1,3-Butadien-1,4-diyl
- oder 4,5-(1,3-Butadien-1,4-diyl), 3-$OCH_3$, H und H sind,

in freier Form oder, wenn diese Formen existieren, in Salzform.

**8.** Verfahren zur Herstellung eines Arzneimittels, umfassend, daß man eine Verbindung der Formel I, wie in Anspruch 1 definiert, in freier Form oder, wenn Salze existieren, in Form eines pharmazeutisch annehmbaren Salzes mit mindestens einem pharmazeutisch annehmbaren Träger oder Verdünnungsmittel vermischt.

**Revendications**

**Revendications pour les Etats contractants suivants : AT, BE, CH, DE, DK, FR, GB, IT, LI, LU, NL, PT, SE**

**1.** Un composé de formule I

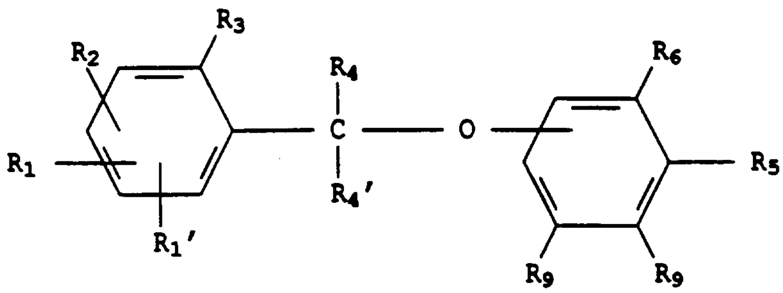

I

dans laquelle

$R_1$, $R_2$ et $R_3$ signifient indépendamment l'hydrogène, un halogène ou un groupe hydroxy, alcoxy, acyloxy ou benzyloxy, deux de ces substituants en positions adjacentes pouvant également former ensemble avec les atomes de carbone auxquels ils sont fixés, un cycle aliphatique, hétéroaliphatique, aromatique ou hétéroaromatique, et

$R_1'$ signifie l'hydrogène, ou bien

$R_1$ et $R_1'$ en positions adjacentes forment, ensemble avec les atomes de carbone auxquels ils sont fixés, un cycle aromatique, et

$R_2$ et $R_3$ sont tels que définis ci-dessus,

$R_4$ et $R_4'$ signifient indépendamment l'hydrogène ou un groupe alkyle, alcényle, cyano, carboxy, alcoxycarbonyle, $-CONR_7R_8$ ou un groupe phényle éventuellement substitué par de l'halogène ou par des groupes hydroxy ou alcoxy, et $R_7$ et $R_8$ signifient indépendamment l'hydrogène ou un groupe alkyle, phényle ou benzyle ou forment, ensemble avec l'atome d'azote auquel ils sont fixés, un cycle saturé à 5 ou 6 chaînons, ou bien

l'un de $R_4$ et $R_4'$ signifie l'hydrogène et l'autre, ensemble avec $R_3$ et les 3 atomes de carbone les séparant, forme un cycle aliphatique, hétéroaliphatique, aromatique ou hétéroaromatique,

$R_5$ signifie un groupe hydroxy, acyloxy, alcoxy, benzyloxy, amino, acylamino ou un groupe de formule

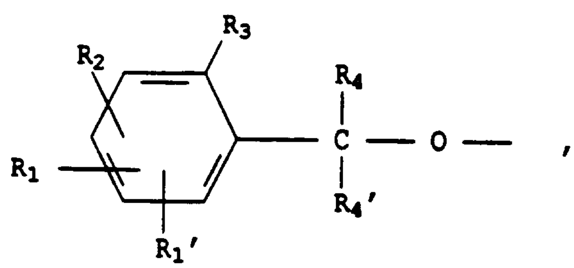

dans laquelle les substituants sont tels que définis plus haut,

$R_6$ signifie un groupe carboxy, alcoxycarbonyle, benzyloxycarbonyle, phénoxycarbonyle, alcényloxycarbonyle, $-CONR_7R_8$ où $R_7$ et $R_8$ sont tels que définis plus haut ou un groupe cyano, et

les deux $R_9$ signifient l'hydrogène ou forment, ensemble avec les atomes de carbone auxquels ils sont fixés, un cycle aromatique,

au moins un de $R_1$, $R_2$ et $R_3$ devant avoir une signification autre que l'hydrogène ou le chlore,
sous forme libre ou, lorsque de telles formes existent, sous forme d'un sel.

2. Un composé selon la revendication 1 de formule I dans laquelle $R_1$, $R_2$ et $R_3$ ont une signification autre qu'un halogène et le reste benzyloxy est en position ortho ou méta par rapport à $R_6$, sous forme libre ou, lorsque de telles formes existent, sous forme d'un sel.

3. Un composé selon la revendication 1 de formule I dans laquelle

| | |
|---|---|
| $R_1$ | signifie l'hydrogène ou un groupe alcoxy en $C_1$-$C_4$ ou signifie, ensemble avec $R_2$, un groupe méthylènedioxy, et |
| $R_1'$ | signifie l'hydrogène, ou bien |
| $R_1$ et $R_1'$ | en positions adjacentes forment, ensemble avec les atomes de carbone auxquels ils sont fixés, un cycle phényle, |
| $R_2$ | signifie un groupe hydroxy, alcoxy en $C_1$-$C_4$ ou acétoxy ou signifie, ensemble avec $R_1$, un groupe méthylènedioxy, |
| $R_3$ | signifie l'hydrogène ou un groupe hydroxy ou alcoxy en $C_1$-$C_4$, |
| $R_4$ et $R_4'$ | signifient l'hydrogène, |
| $R_5$ | signifie un groupe hydroxy, acétoxy, alcoxy en $C_1$-$C_4$, amino, acétylamino, 2,5-diméthoxybenzyloxy ou 3-acétoxybenzyloxy, |
| $R_6$ | signifie un groupe carboxy, alcoxycarbonyle en $C_2$-$C_5$, cyano ou $-CONR_7R_8$ où $R_7$ et $R_8$ signifient indépendamment l'hydrogène ou un groupe alkyle en $C_1$-$C_4$ ou benzyle ou forment, ensemble avec l'atome d'azote auquel ils sont fixés, un groupe pipéridino, |
| les deux $R_9$ | signifient l'hydrogène ou forment, ensemble avec les atomes de carbone auxquels ils sont fixés, un cycle phényle, |

au moins un de $R_1$, $R_2$ et $R_3$ devant avoir une signification autre que l'hydrogène, sous forme libre ou, lorsque de telles formes existent, sous forme d'un sel.

4. Un composé selon la revendication 1 de formule I dans laquelle

| | |
|---|---|
| $R_1$, $R_2$, $R_3$ et $R_4$ | sont tels que définis à la revendication 1 sous la formule I, |
| $R_1'$ et $R_9$ | signifient l'hydrogène, |
| $R_5$ | signifie un groupe hydroxy, benzyloxy, alcoxy ou acyloxy, et |
| $R_6$ | à l'exception du groupe carboxy et cyano, a la signification indiquée à la revendication sous la formule I, |

au moins un de $R_1$, $R_2$ et $R_3$ devant avoir une signification autre que l'hydrogène ou le chlore, sous forme libre ou, lorsque de telles formes existent, sous forme d'un sel.

5. Un composé selon la revendication 1 qui est l'ester méthylique de l'acide 5-(2,5-diméthoxybenzyloxy)-2-hydroxybenzoïque.

6. Un composé selon la revendication 1, qui est
- l'ester méthylique de l'acide 5-(2,5-dihydroxybenzyloxy)-2-hydroxybenzoïque,
- l'acide 5-(2,5-diméthoxybenzyloxy)-2-hydroxybenzoïque,
- l'amide de l'acide 5-(2,5-diméthoxybenzyloxy)-2-hydroxybenzoïque,
- le 5-(2,5-diméthoxybenzyloxy)-2-hydroxybenzonitrile,
- l'ester méthylique de l'acide 5-(2,5-diméthoxybenzyloxy)-2-méthoxybenzoïque,
- l'ester méthylique de l'acide 5-(2,5-diméthoxybenzyloxy)-2-acétoxybenzoïque, ou bien
- de formule I dans laquelle $R_1'$, $R_1$, $R_4$, $R_4'$ et les deux $R_9$ signifient H et $R_2$, $R_3$, $R_5$, $R_6$ et la position du reste benzyloxy par rapport à $R_6$ signifient respectivement
  - 5-$OCH_3$, $OCH_3$, OH, $COOCH_3$ et 4,
  - 3-$OCH_3$, $OCH_3$, OH, $COOCH_3$ et 5,
  - 3-$OCH_3$, $OCH_3$, OH, $COOCH_3$ et 4,
  - 5-$OCH_3$, $OCH_3$, OH, $COOCH_3$ et 3,
  - 5-$OCH_3$, $OCH_3$, OH, $COOCH_3$ et 6,
  - 5-$OCH_3$, $OCH_3$, $NHCOCH_3$, $COOCH_3$ et 5,
  - 5-$OCH_3$, $OCH_3$, $NH_2$, $COOCH_3$, et 5,
  - 3-OH, H, OH, $COOCH_3$ et 5,
  - 5-$OCH_3$, OH, OH, $COOCH_3$ et 5,
  - 5-$OCH_3$, $OCH_3$, OH, $CONHCH_2C_6H_5$ et 5,
  - 5-$OCH_3$, $OCH_3$, OH, 1-pipéridylcarbonyle et 5,
  - 5-$OCH_3$, $OCH_3$, OH, $CONHCH_3$ et 5,

- 5-$OCH_3$, $OCH_3$, 2,5-di-$OCH_3$-benzyloxy, $COOCH_3$ et 5,
- 3-$OCOH_3$, H, 3-acétoxybenzyloxy, $COOCH_3$ et 5,
- 3-$OCOH_3$, H, OH, $COOCH_3$ et 5, ou bien

- de formule I dans laquelle $R_1$', $R_4$, $R_4$' et les deux $R_9$ signifie H, $R_5$ signifie OH, $R_6$ signifie $COOCH_3$, la position du reste benzyloxy par rapport à $R_6$ est 5 et $R_1$, $R_2$, $R_3$ signifient respectivement,
  - 3,4-$OCH_2O$- pour $R_1$ et $R_2$ et H pour $R_3$, ou bien
  - 4-$OCH_3$, 3-$OCH_3$ et $OCH_3$, ou bien
- de formule I dans laquelle $R_3$ signifie $OCH_3$, $R_4$ et $R_4$' signifient H, $R_5$ signifie OH, $R_6$ signifie $COOCH_3$, la position du reste benzyloxy par rapport à $R_6$ est 5 et $R_1$', $R_1$, $R_2$ et les deux $R_9$ signifient respectivement,
  - H, H, 5-$OCH_3$ et 1,3-butadiène-1,4-diyle, ou bien
  - 4,5-(1,3-butadiène-1,4-diyle), 3-$OCH_3$, H et H,

sous forme libre ou, lorsque de telles formes existent, sous forme d'un sel.

**7.** Un procédé de préparation d'un composé selon la revendication 1, comprenant la réaction d'un composé de formule II

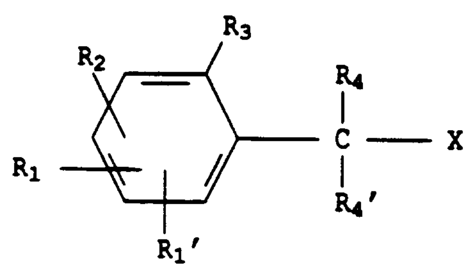

II

dans laquelle
$R_1$, $R_1$', $R_2$, $R_3$, $R_4$ et $R_4$' sont tels que définis à la revendication 1 et X signifie un groupe éliminable, avec un composé de formule III

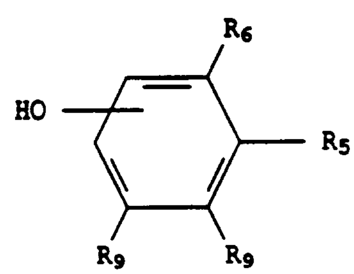

III

dans laquelle les substituants sont tels que définis plus haut,
sous forme protégée lorsque cela est approprié, et
lorsqu'un composé résultant est sous forme protégée, l'élimination de façon appropriée du ou des groupes protecteurs de ce composé, et/ou la transformation d'un composé résultant de formule I en un autre composé de formule I, et la récupération des composés ainsi obtenus sous forme libre ou, lorsque de telles formes existent, sous forme d'un sel.

**8.** Un procédé selon la revendication 7, qui est effectué en présence de triphénylphosphine et d'un ester alkylique de l'acide azodicarbonique.

**9.** Un composé selon la revendication 1 sous forme libre ou, lorsque les sels existent, sous forme d'un sel pharmaceutiquement acceptable, pour l'utilisation comme médicament, spécialement pour le traitement des troubles hyperprolifératifs/inflammatoires et du cancer.

**10.** Une composition pharmaceutique comprenant un composé selon la revendication 1, sous forme libre ou, lorsque les sels existent, sous forme d'un sel pharmaceutiquement acceptable, ensemble avec au moins un véhicule ou diluant pharmaceutiquement acceptable.

**Revendications pour les Etats contractants suivants : ES, GR**

**1.** Un procédé de préparation d'un composé de formule I

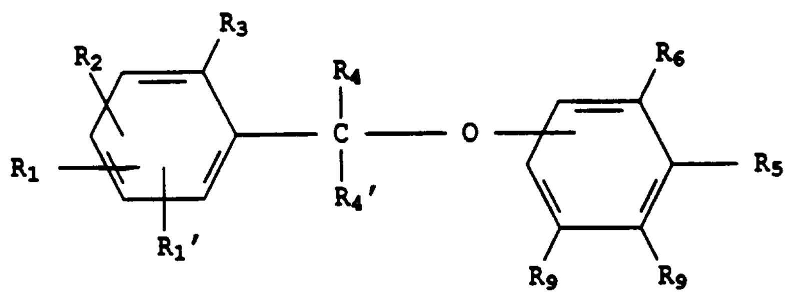

I

dans laquelle

$R_1$, $R_2$ et $R_3$ signifient indépendamment l'hydrogène, un halogène ou un groupe hydroxy, alcoxy, acyloxy ou benzyloxy, deux de ces substituants en positions adjacentes pouvant également former, ensemble avec les atomes de carbone auxquels ils sont fixés, un cycle aliphatique, hétéroaliphatique, aromatique ou hétéroaromatique, et

$R_1$' signifie l'hydrogène, ou bien

$R_1$ et $R_1$' en positions adjacentes forment, ensemble avec les atomes de carbone auxquels ils sont fixés, un cycle aromatique, et

$R_2$ et $R_3$ sont tels que définis ci-dessus,

$R_4$ et $R_4$' signifient indépendamment l'hydrogène ou un groupe alkyle, alcényle, cyano, carboxy, alcoxycarbonyle, $-CONR_7R_8$ ou un groupe phényle éventuellement substitué par de l'halogène ou par des groupes hydroxy ou alcoxy, et $R_7$ et $R_8$ signifient indépendamment l'hydrogène ou un groupe alkyle, phényle ou benzyle ou forment, ensemble avec l'atome d'azote auquel ils sont fixés, un cycle saturé à 5 ou 6 chaînons, ou bien

l'un de $R_4$ et $R_4$' signifie l'hydrogène et l'autre, ensemble avec $R_3$ et les 3 atomes de carbone les séparant, forme un cycle aliphatique, hétéroaliphatique, aromatique ou hétéroaromatique,

$R_5$ signifie un groupe hydroxy, acyloxy, alcoxy, benzyloxy, amino, acylamino ou un groupe de formule

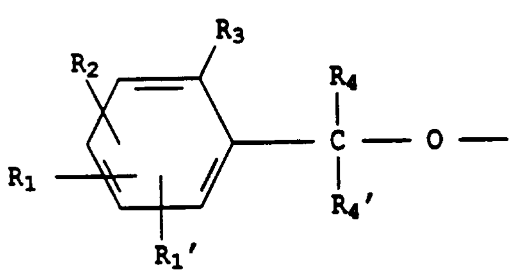

,

dans laquelle les substituants sont tels que définis plus haut,

$R_6$ signifie un groupe carboxy, alcoxycarbonyle, benzyloxycarbonyle, phénoxycarbonyle, alcényloxycarbonyle, $-CONR_7R_8$ où $R_7$ et $R_8$ sont tels que définis plus haut ou un groupe cyano, et

les deux $R_9$ signifient l'hydrogène ou forment, ensemble avec les atomes de carbone auxquels ils sont fixés, un cycle aromatique,

au moins un de $R_1$, $R_2$ et $R_3$ devant avoir une signification autre que l'hydrogène ou le chlore,
sous forme libre ou, lorsque de telles formes existent, sous forme d'un sel,

lequel procédé comprend la réaction d'un composé de formule II

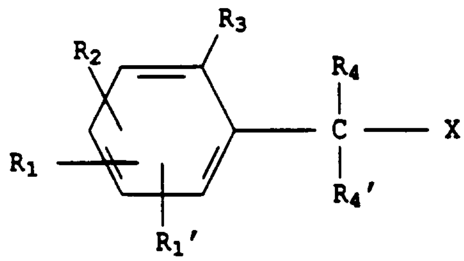

II

dans laquelle
$R_1$, $R_1$', $R_2$, $R_3$, $R_4$ et $R_4$' sont tels que définis dans cette revendication et X signifie un groupe éliminable,
avec un composé de formule III

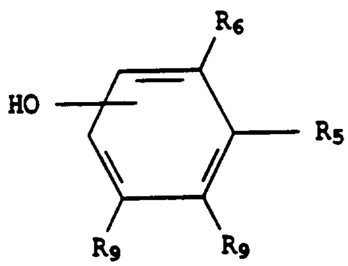

III

dans laquelle les substituants sont tels que définis dans cette revendication,
sous forme protégée lorsque cela est approprié, et
lorsqu'un composé résultant est sous forme protégée, l'élimination de façon appropriée du ou des groupes protecteurs de ce composé, et/ou la transformation d'un composé résultant de formule I en un autre composé de formule I, et la récupération des composés ainsi obtenus sous forme libre ou, lorsque de telles formes existent, sous forme d'un sel.

**2.** Un procédé selon la revendication 1 qui est effectué en présence de triphénylphosphine et d'un ester alkylique de l'acide azodicarbonique.

**3.** Un procédé selon la revendication 1 ou 2 pour la préparation d'un composé selon la revendication 1 de formule I dans laquelle $R_1$, $R_2$ et $R_3$ ont une signification autre qu'un halogène et le reste benzyloxy est en position ortho ou méta par rapport à $R_6$, sous forme libre ou, lorsque de telles formes existent, sous forme d'un sel.

**4.** Un procédé selon la revendication 1 ou 2 pour la préparation d'un composé de formule I dans laquelle

| | |
|---|---|
| $R_1$ | signifie l'hydrogène ou un groupe alcoxy en $C_1$-$C_4$ ou signifie, ensemble avec $R_2$, un groupe méthylènedioxy, et |
| $R_1$' | signifie l'hydrogène, ou bien |
| $R_1$ et $R_1$' | en positions adjacentes forment, ensemble avec les atomes de carbone auxquels ils sont fixés, un cycle phényle, |
| $R_2$ | signifie un groupe hydroxy, alcoxy en $C_1$-$C_4$ ou acétoxy ou signifie, ensemble avec $R_1$, un groupe méthylènedioxy, |
| $R_3$ | signifie l'hydrogène ou un groupe hydroxy ou alcoxy en $C_1$-$C_4$, |
| $R_4$ et $R_4$' | signifient l'hydrogène, |
| $R_5$ | signifie un groupe hydroxy, acétoxy, alcoxy en $C_1$-$C_4$, amino, acétylamino, 2,5-diméthoxybenzyloxy ou 3-acétoxybenzyloxy, |
| $R_6$ | signifie un groupe carboxy, alcoxycarbonyle en $C_2$-$C_5$, cyano ou $-CONR_7R_8$ où $R_7$ et $R_8$ signifient indépendamment l'hydrogène ou un groupe alkyle en $C_1$-$C_4$ ou benzyle ou forment, ensemble avec l'atome d'azote auquel ils sont fixés, un groupe |

pipéridino,

les deux $R_9$ signifient l'hydrogène ou forment, ensemble avec les atomes de carbone auxquels ils sont fixés, un cycle phényle,

au moins un de $R_1$, $R_2$ et $R_3$ devant avoir une signification autre que l'hydrogène, sous forme libre ou, lorsque de telles formes existent, sous forme d'un sel.

**5.** Un procédé selon la revendication 1 ou 2 pour la préparation d'un composé selon la revendication 1 de formule I dans laquelle
$R_1$, $R_2$, $R_3$ et $R_4$ sont tels que définis à la revendication 1 sous la formule I,
$R_1$' et $R_9$ signifient l'hydrogène,
$R_5$ signifie un groupe hydroxy, benzyloxy, alcoxy ou acyloxy, et
$R_6$ à l'exception du groupe carboxy et cyano, a la signification indiquée à la revendication 1 sous la formule I,
au moins un de $R_1$, $R_2$ et $R_3$ devant avoir une signification autre que l'hydrogène ou le chlore,
sous forme libre ou, lorsque de telles formes existent, sous forme d'un sel.

**6.** Un procédé selon la revendication 1 ou 2 pour la préparation de l'ester méthylique de l'acide 5-(2,5-diméthoxybenzyloxy)-2-hydroxybenzoïque.

**7.** Un procédé selon la revendication 1 ou 2 pour la préparation d'un composé selon la revendication 1, qui est
- l'ester méthylique de l'acide 5-(2,5-dihydroxybenzyloxy)-2-hydroxybenzoïque,
- l'acide 5-(2,5-diméthoxybenzyloxy)-2-hydroxybenzoïque,
- l'amide de l'acide 5-(2,5-diméthoxybenzyloxy)-2-hydroxybenzoïque,
- le 5-(2,5-diméthoxybenzyloxy)-2-hydroxybenzonitrile,
- l'ester méthylique de l'acide 5-(2,5-diméthoxybenzyloxy)-2-méthoxybenzoïque,
- l'ester méthylique de l'acide 5-(2,5-diméthoxybenzyloxy)-2-acétoxybenzoïque, ou bien
- de formule I dans laquelle $R_1$', $R_1$, $R_4$, $R_4$' et les deux $R_9$ signifient H et $R_2$, $R_3$, $R_5$, $R_6$ et la position du reste benzyloxy par rapport à $R_6$ signifient respectivement
  - 5-$OCH_3$, $OCH_3$, OH, $COOCH_3$ et 4,
  - 3-$OCH_3$, $OCH_3$, OH, $COOCH_3$ et 5,
  - 3-$OCH_3$, $OCH_3$, OH, $COOCH_3$ et 4,
  - 5-$OCH_3$, $OCH_3$, OH, $COOCH_3$ et 3,
  - 5-$OCH_3$, $OCH_3$, OH, $COOCH_3$ et 6,
  - 5-$OCH_3$, $OCH_3$, $NHCOCH_3$, $COOCH_3$ et 5,
  - 5-$OCH_3$, $OCH_3$, $NH_2$, $COOCH_3$ et 5,
  - 3-OH, H, OH, $COOCH_3$ et 5,
  - 5-$OCH_3$, OH, OH, $COOCH_3$ et 5,
  - 5-$OCH_3$, $OCH_3$, OH, $CONHCH_2$-$C_6H_5$ et 5,
  - 5-$OCH_3$, $OCH_3$, OH, 1-pipéridylcarbonyle et 5,
  - 5-$OCH_3$, $OCH_3$, OH, $CONHCH_3$ et 5,
  - 5-$OCH_3$, $OCH_3$, 2,5-di-$OCH_3$-benzyloxy, $COOCH_3$ et 5,
  - 3-$OCOH_3$, H, 3-acétoxybenzyloxy, $COOCH_3$ et 5,
  - 3-$OCOH_3$, H, OH, $COOCH_3$ et 5, ou bien
- de formule I dans laquelle $R_1$', $R_4$, $R_4$' et les deux $R_9$ signifie H, $R_5$ signifie OH, $R_6$ signifie $COOCH_3$, la position du reste benzyloxy par rapport à $R_6$ est 5 et $R_1$, $R_2$, $R_3$ signifient respectivement,
  - 3,4-$OCH_2O$- pour $R_1$ et $R_2$ et H pour $R_3$, ou bien
  - 4-$OCH_3$, 3-$OCH_3$ et $OCH_3$, ou bien
- de formule I dans laquelle $R_3$ signifie $OCH_3$, $R_4$ et $R_4$' signifient H, $R_5$ signifie OH, $R_6$ signifie $COOCH_3$, la position du reste benzyloxy par rapport à $R_6$ est 5 et $R_1$', $R_1$, $R_2$ et les deux $R_9$ signifient respectivement,
  - H, H, 5-$OCH_3$ et 1,3-butadiène-1,4-diyle, ou bien
  - 4,5-(1,3-butadiène-1,4-diyle), 3-$OCH_3$, H et H,

sous forme libre ou, lorsque de telles formes existent, sous forme d'un sel.

**8.** Un procédé de préparation d'un médicament qui comprend le mélange d'un composé de formule I tel que défini à la revendication 1, sous forme libre ou, lorsque les sels existent, sous forme d'un sel

pharmaceutiquement acceptable, avec au moins un véhicule ou diluant pharmaceutiquement acceptable.